# EUROPEAN PATENT APPLICATION

(11) **EP 2 382 919 A1**
(43) Date of publication of application: **02.11.2011**
(21) Application number: 11167369.5
(22) Date of filing: 30.03.2007
(51) Int. Cl.: A61B 5/00, H04B 3/00

(54) **Glucose meter communication method and system**

(30) Priority: 03.04.2006 US 397937
(62) Divisional of application: 07754727.1
(71) Applicant: Cardiocom, LLC, Chanhassen MN 55317 (US)
(72) Inventor: Cosentino, Daniel, L., Chaska, MN 55318 (US); Cosentino, Louis, C., Excelsior, MN 55331 (US); Golden, Brian, Alan, Eden Prairie, MN 55347 (US)
(74) Representative: Hassa, Oliver Michael

(57) **Abstract**

Methods for communicating between a glucose meter and a computing system are described. One method includes automatically initiating a communication session with a computing system over a communication link. The method also includes automatically sending data from the glucose meter to the computing system via the communication link.

## Description

This application is being filed on 29 March 2007, as a PCT International Patent application in the name of Cardiocom, LLC, a U.S. national corporation, applicant for the designation of all countries except the US, and Daniel L. Cosentino, Louis C. Cosentino, and Brian Alan Golden, citizens of the U.S., applicants for the designation of the US only, and claims priority to U.S. Utility Patent Application Serial No. 11/397,937, filed April 3, 2006.

### Technical Field

The present invention is related to communication of wellness parameters for remote patient monitoring; in particular, the present invention is related to methods and systems for communicating wellness parameters.

### Background

The incidence of diabetes mellitus is increasing rapidly in developed countries due to increasing obesity, inactive lifestyles and an aging population. Estimates by the World Health Organization have shown the current global prevalence of diabetes is 3% (194 million people) and is expected to increase in prevalence to 6.3% by 2025. As the incidence of diabetes increases, a corresponding increase in diabetes monitoring and care will be needed.

The goal of any type of diabetes care is to keep blood glucose levels as normal as possible. Complications of diabetes may be more prevalent if blood glucose is not controlled. Some examples of complications are high blood pressure, stroke, eye disease / blindness, kidney disease, heart disease, foot disease and amputations, complications of pregnancy, skin and dental disease. In order to keep blood glucose levels normal, diabetics require regular feedback regarding their current blood glucose levels. This will provide guidance on how to improve future readings, thereby providing a positive educational experience that will influence their long term health.

Most diabetics use glucose meters to check their blood glucose. To test glucose levels with a typical meter, blood is placed on a disposable test strip and placed in the meter. The test strips are coated with suitable chemicals, such as glucose oxidase, dehydrogenase, or hexokinase, that combine with glucose in the blood. The meter measures how much glucose is present based on the reactions with these chemicals.

Most glucose meters contain a portal in which the meter can communicates with another device such as Infrared (IR), bluetooth, wireless, and wired ports that can be used to manually download glucose readings to a PC or other remote patient monitoring devices, such as the Cardiocom® Commander device. The remote patient monitoring device can then store and compare a large number of test results, and communicate these test results to a health care provider that is monitoring the diabetic patient. However, the method and process of such communication can be difficult and often complex for the users of blood glucose meters.

In addition to communication barriers, most glucose meters are battery powered, the frequency and duration of communication sessions with other devices can be limited secondary to the life of the battery. Due to power constraints, glucose meters usually require manual intervention by the user to start a communication session. The manual processes required to communicate with external PC's and other remote monitoring devices are usually cumbersome and complex for users, and therefore the frequency with which communication between meter, monitoring device, and health care provider can be low .

Health care providers monitoring diabetic patients need to have access to blood glucose test results in order to determine if the patient is on the correct treatment, and after studying these glucose readings adjust the regimen accordingly. When diabetic patients do not regularly provide test results because of technical complexity, physical communication constraints or complacence, the health care provider's ability to provide proper care is limited Diabetic patients may want to review their blood glucose test results. These patients would want access to complete records of test results as well, rather than only those which they remembered to record

Patients have further concerns regarding the disposable test strips used in glucose meters. Characteristics of the disposable test strips can vary from one supply to another. For example, the concentration of glucose oxidase, dehydrogenase, or hexokinase can vary between test strip supplies, which can result in varied readings from the glucose meter. Current glucose meters accept a manually entered code or microchip that allow the glucose meter to calibrate its reading to the particular test strips being used. Diabetics often forget to replace the microchip or otherwise recalibrate their glucose meters when changing supplies of test strips, leading to inaccurate blood glucose test results.

For these and other reasons, improvements are desirable.

### Summary

In accordance with the present invention, the above and other problems are solved by the following:
In a first aspect, a method of communicating data between a glucose meter and a computing system is disclosed. The method includes automatically initiating a communication session between the glucose meter and the computing system over a communication link. The method further includes automatically sending data from the glucose meter to the computing system via the communication link.
In a second aspect, a method of gathering and communicating test results with a glucose meter is disclosed. The method includes storing a predetermined communication time. The method further includes initiating the glucose meter to enter a low power mode. The system includes initiating a glucose meter to exit the low power mode upon reaching the predetermined communcation time. The system includes automatically initiating a communication session with a computing system via a communication link at the predetermined communication time. The system includes sending the test result to the computing system via the communication link.
In a third aspect, a method of obtaining blood glucose test results from a glucose meter is disclosed. The method includes repetitively listening for a signal from a glucose meter on a communication link at a given frequency. The method further includes detecting the existence of a response from a glucose meter on the communication link. The method includes initiating communication with the glucose meter via the communication link. The method includes receiving a response from a glucose meter via the communication link.

In yet another aspect, a system for coordinating communication of blood glucose test results is disclosed. The system includes a glucose meter configured to obtain a test result representative of a blood glucose level of a patient. The system also includes a remote system configured to communicate with the glucose meter via a network. The remote system is configured to receive and store test results obtained by the glucose meter in a database containing patient data. The remote system periodically receives the one or more test results via a communication session automatically initiated by a component of the system. The remote system also tracks the glucose level of the patient and generates an alert if the test result is outside parameters set by a health care provider.

According to yet another aspect, a glucose meter system including a glucose meter and a line powered communication device is disclosed. The glucose meter is configured to determine a test result representative of a patient's blood glucose level. The line powered communication device is communicatively coupled to the glucose meter, and is configured to access data and communicate the data over a wired communcation link. The line powered device is at least partially powered with power received from the wired communication link. The data includes the test result.

According to a further aspect, a blood glucose test result communication system is disclosed. The system includes a glucose meter configured to compute and store a test result representative of a patient's blood glucose level. The systems also includes a line powered communication device communicatively coupled to the glucose meter and configured to automatically access the test result and communicate the test result over a wired communications link, wherein the line powered communication device is at least partially powered with power received from the wired communication link, The system further includes a remote system communicatively connected to the line powered communication device via the communications link. The remote system is configured to accept and store the test result in a database.

According to a further aspect, a method of communicating between a glucose meter and a remote system is disclosed, wherein the glucose meter is communicatively connected to a line powered communication device at least partially powered with power received from a wired communication link. The method includes acquiring a test result representative of a blood glucose level, the test result accessible to the glucose meter and the line powered communication device. The method includes placing the glucose meter in a low power mode. The method further includes transferring the test result from the line powered communication device to the remote system.

According to yet another aspect, a method of communicating a blood glucose test result between a glucose meter and a computing system is disclosed. The method includes waking the glucose meter from a low power state. The method includes obtaining a blood glucose test result. The method also includes storing a predetermined communication time. The method includes physically connecting the glucose meter to a communications device. The mehtod includes placing the glucose meter in the low power state. The method also includes automatically initiating a communication session between the glucose meter and the computing system over a communication link at the predetermined communication time and in response to physically connecting the glucose meter to the communications device.

### Brief Description of the Drawings

FIG. 1 is a schematic representation of a blood glucose monitoring system according to an example embodiment of the present disclosure;
FIG. 2 is a schematic representation of a computing system that can be used to implement aspects of the present disclosure;
FIG. 3 is a schematic representation of a blood glucose monitoring system according to an example embodiment of the present disclosure;
FIG. 4 is a schematic representation of a blood glucose monitoring system according to an example embodiment of the present disclosure;
FIG. 5 is a schematic representation of a monitoring system that can be used to implement aspects of the present disclosure;
FIG. 6 depicts a physical structure of a monitoring system usable by multiple users according to an example embodiment of the present disclosure;
FIG. 7 depicts a physical structure of a monitoring system usable by multiple users according to an example embodiment of the present disclosure;
FIG. 8 is a schematic representation of a glucose meter within a monitoring system that can be used to implement aspects of the present disclosure;
FIG. 9 is a schematic representation of a glucose meter within a monitoring system that can be used to implement further aspects of the present disclosure;
FIG. 10 is a connection diagram of a portion of a blood glucose monitoring system according to an example embodiment of the present disclosure;
FIG. 11 is a schematic view of a communications device according to an example embodiment of the present disclosure;
FIG. 12 is a schematic representation of a communications device according to an example embodiment of the present disclosure;
FIG. 13 is an electrical schematic of internal circuitry for a glucose meter according to an example embodiment of the present disclosure;
FIG. 14A is a schematic representation of a portion of a glucose meter incorporating a line-powered modem according to an example embodiment of the present disclosure;
FIG. 14B is a schematic representation of a portion of a glucose meter incorporating a line-powered modem according to an example embodiment of the present disclosure;
FIG. 15 is a schematic representation of a glucose meter accepting a test strip according to an example embodiment of the present disclosure;
FIG. 16 is a schematic representation of a glucose meter accepting a test strip according to an example embodiment of the present disclosure;
FIG. 17 is a flow diagram of systems and methods for blood glucose monitoring according to an example embodiment of the present disclosure;
FIG. 18 is a flow diagram of systems and methods for blood glucose monitoring according to an example embodiment of the present disclosure;
FIG. 19 is a sample exception report generated according to an example embodiment of the present disclosure;
FIG. 20 is a flow diagram of systems and methods for communicating data in a glucose meter according to a possible embodiment of the present disclosure;
FIG. 21 is a flow diagram of systems and methods for communicating data in a glucose meter according to a possible embodiment of the present disclosure;
FIG. 22 is a flow diagram of systems and methods for communicating data in a glucose meter according to a possible embodiment of the present disclosure;
FIG. 23 is a flow diagram of systems and methods for blood glucose monitoring according to an example embodiment of the present disclosure;
FIG. 24 is a flow diagram of systems and methods for calibration and use of a glucose meter according to an example embodiment of the present disclosure;
FIG. 25 is a flow diagram of a system for controlling a glucose meter and line-powered communications device according to a possible embodiment;
FIG. 26 is a flow diagram of a data connection system for use in conjunction with a glucose meter according to an example embodiment of the present disclosure;
FIG. 27 is a flow diagram of a system for glucose meter communication is shown according to an example embodiment of the present disclosure; and
FIG. 28 is a flow diagram of a system for glucose meter communication is shown according to an example embodiment of the present disclosure.

### Detailed Description

In general, the present disclosure is related to improved glucose test result communication to health care providers and patients. Various methods and systems disclosed herein provide the structural and functional aspects used to accomplish the goal of easier, simpler communication of and access to accurate glucose meter data. The improved glucose meter communication is generally accomplished by automation and streamlining of specific tasks that typically require manual intervention of either the diabetic patient or health care provider.

Automating communications between a glucose meter and a computing system tightens the communication link between patients and health care providers. This provides a number of advantages for both groups. Automatic communication of at least the status of the glucose meter or blood glucose test results simplifies the blood glucose monitoring task for the patient. Steps are removed from the blood glucose monitoring regimen, allowing for easier compliance by patients. Likewise, communication of this same data allows both health care providers and patients to easily monitor patient compliance with a health care regimen.

As used in the present disclosure, automatic actions are intended to encompass initiating or performing a process or processes without the need for user intervention. Where a specific function, module, or method step is performed automatically following a user-performed step, it is intended that no additional user intervention is required. However, it is not intended that the function, module, or method step occurs immediately upon occurrence of an event, although in various implementations that may be true. Specific automatic techniques described herein include establishing communication sessions between electronic devices, data transmission, and mechanical or electrical interactions occurring, for example, on preprogrammed devices. The present disclosure is not limited to automation of these techniques, as other techniques may be automated consistent with this disclosure.

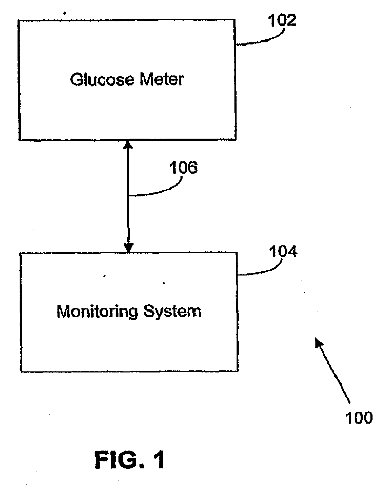

Referring now to FIG. 1, a schematic representation of a blood glucose monitoring system 100 is shown according to the present disclosure. The blood glucose monitoring system 100 includes both a glucose meter 102 and a monitoring system 104. The blood glucose monitoring system 100 is configured to provide tighter communication between a patient, the patient's glucose meter 102, and a monitoring system 104 configured to track glucose meter activity and glucose test results as reported by the glucose meter 102. A communication link 106 can be used between the glucose meter 102 and the monitoring system 104 to communicate data from the glucose meter, which can include blood glucose test results.

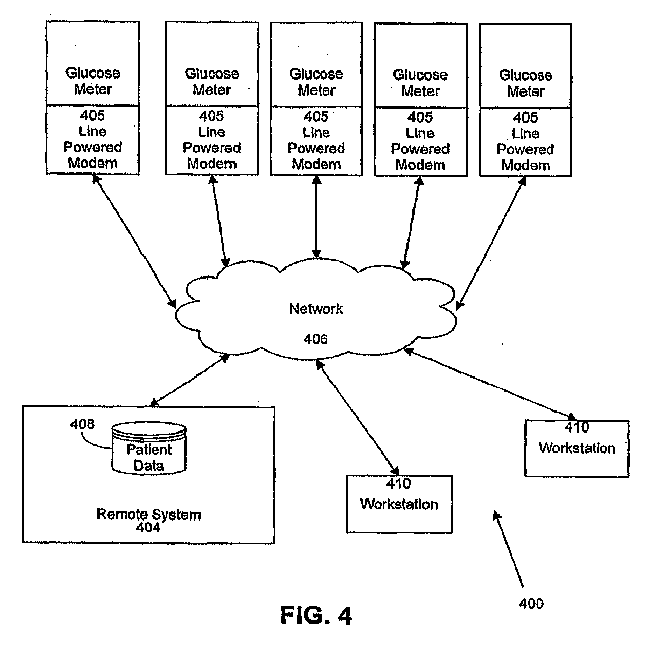

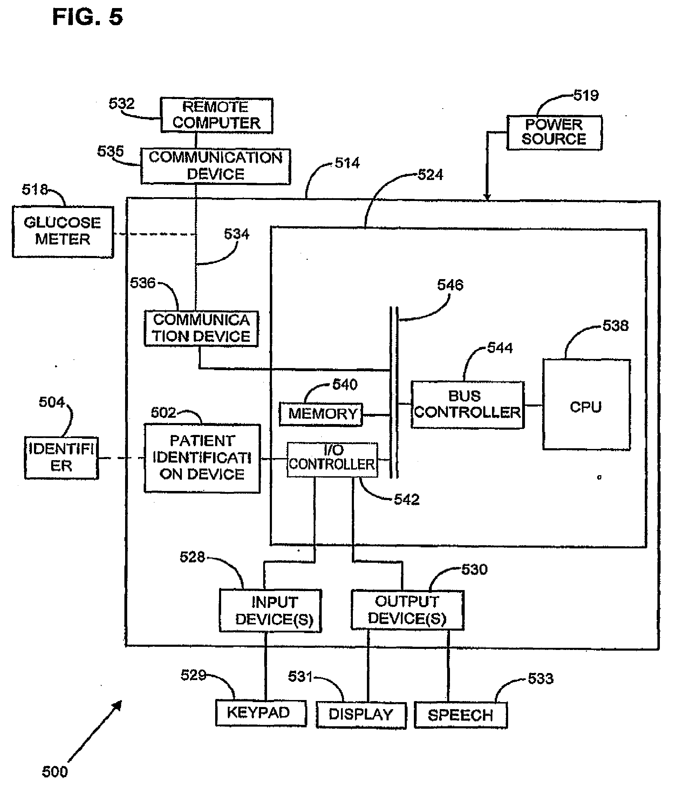

The glucose meter 102 can be any of a number of configurations of glucose meters, and in certain aspects of the present disclosure additional features are discussed herein as having certain advantageous properties. Such glucose meters will typically receive glucose test strips and also have a communication device integrated so as to connect to the monitoring system. Two examples of possible glucose meters according to the present disclosure are shown below in conjunction with FIGS. 4 or 5.

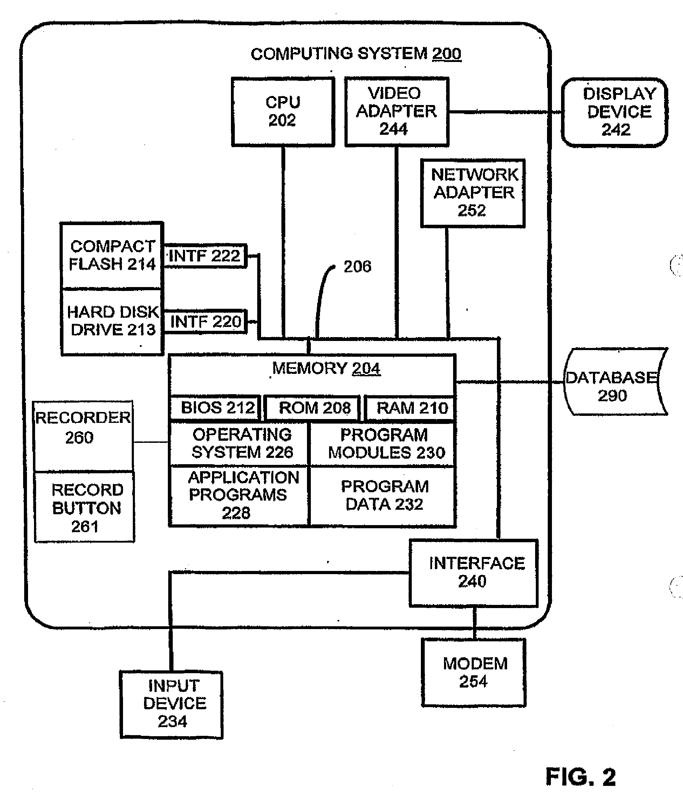

The monitoring system 104 is preferably configured to store blood glucose test results that are received from the glucose meter. In certain aspects, the monitoring system 104 can be any of a number of general or specialized computing systems, such as those shown below in conjunction with FIGS. 2-7. The communication link 106 is a data communication link that can be wired or wireless, and can use any of a number of communication protocols.

Referring now to FIG. 2, an exemplary environment for implementing embodiments of the present invention includes a general purpose computing device in the form of a computing system 200, including at least one processing system 202. A variety of processing units are available from a variety of manufacturers, for example, Intel or Advanced Micro Devices. The computing system 200 also includes a system memory 204, and a system bus 206 that couples various system components including the system memory 204 to the processing unit 202. The system bus 206 may be any of a number of types of bus structures including a memory bus, or memory controller; a peripheral bus; and a local bus using any of a variety of bus architectures.

Preferably, the system memory 204 includes read only memory (ROM) 208 and random access memory (RAM) 210. A basic input/output system 2l2 (BIOS), containing the basic routines that help transfer information between elements within the computing system 200, such as during start-up, is typically stored in the ROM 208.

Preferably, the computing system 200 further includes a secondary storage device 213, such as a hard disk drive, for reading from and writing to a hard disk (not shown), and/or a compact flash card 214.

The hard disk drive 213 and compact flash card 214 are connected to the system bus 206 by a hard disk drive interface 220 and a compact flash card interface 222, respectively. The drives and cards and their associated computer-readable media provide nonvolatile storage of computer readable instructions, data structures, program modules and other data for the computing system 200.

Although the exemplary environment described herein employs a hard disk drive 213 and a compact flash card 214, it should be appreciated by those skilled in the art that other types of computer-readable media, capable of storing data, can be used in the exemplary system. Examples of these other types of computer-readable mediums include magnetic cassettes, flash memory cards, digital video disks, Bernoulli cartridges, CD ROMS, DVD ROMS, random access memories (RAMs), read only memories (ROMs), and the like.

A number of program modules may be stored on the hard disk 213, compact flash card 214, ROM 208, or RAM 210, including an operating system 226, one or more application programs 228, other program modules 230, and program data 232. A user may enter commands and information into the computing system 200 through an input device 234. Examples of input devices might include a keyboard, mouse, microphone, joystick, game pad, satellite dish, scanner, digital camera, touch screen, and a telephone. These and other input devices are often connected to the processing unit 202 through an interface 240 that is coupled to the system bus 206. These input devices also might be connected by any number of interfaces, such as a parallel port, serial port, game port, or a universal serial bus (USB). A display device 242, such as a monitor or touch screen LCD panel, is also connected to the system bus 206 via an interface, such as a video adapter 244. The display device 242 might be internal or external. In addition to the display device 242, computing systems, in general, typically include other peripheral devices (not shown), such as speakers, printers, and palm devices.

When used in a LAN networking environment, the computing system 200 is connected to the local network through a network interface or adapter 252. When used in a WAN networking environment, such as the Internet, the computing system 200 typically includes a modem 254 or other means, such as a direct connection, for establishing communications over the wide area network. The modem 254, which can be interval or external, is connected to the system bus 206 via the interface 240. In a networked environment, program modules depicted relative to the computing system 200, or portions thereof, may be stored in a remote memory storage device. It will be appreciated that the network connections shown are exemplary and other means of establishing a communication link between the computing systems may be used.

The computing system 200 might also include a recorder 260 connected to the memory 204. The recorder 260 includes a microphone for receiving sound input and is in communication, with the memory 204 for buffering and storing the sound input. Preferably, the recorder 260 also includes a record button 261 for activating the microphone and communicating the sound input to the memory 204.

A computing device, such as computing system 200, typically includes at least some form of computer-readable media. Computer readable media can be any available media that can be accessed by the computing system 200. By way of example, and not limitation, computer-readable media might comprise computer storage media and communication media.

Computer storage media includes volatile and nonvolatile, removable and non-removable media implemented in any method or technology for storage of information such as computer readable instructions, data structures, program modules or other data. Computer storage media includes, but is not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium that can be used to store the desired information and that can be accessed by the computing system 200.

Communication media typically embodies computer-readable instructions, data structures, program modules or other data in a modulated data signal such as a carrier wave or other transport mechanism and includes any information delivery media. The term modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared, and other wireless media. Combinations of any of the above should also be included within the scope of computer-readable media. Computer-readable media may also be referred to as computer program product.

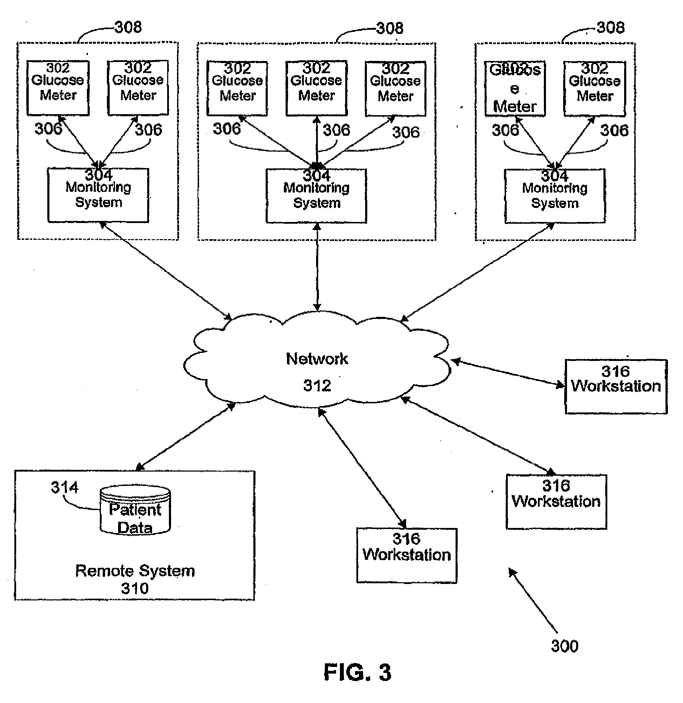

Referring now to FIG. 3, a blood glucose monitoring system 300 is shown according to a possible embodiment of the present disclosure. Generally, the blood glucose monitoring system 300 is arranged and configured such that the various devices incorporated into the system 300 can easily intercommunicate over a common interface, as described in more detail below.

The blood glucose monitoring system 300 includes a number of glucose meters 302 connected to, or incorporated within, monitoring systems 304 over a communication link 306. Generally, the glucose meter 302 and the monitoring system 304 will be at the same location 308, and the communication link 306 can be a wired or wireless communication link requiring little power for operation. For example, the communication link 306 can be a Bluetooth, IrDA, Universal Serial Bus, RS-232, power line networking, or other local networking link. Such systems are particularly advantageous for low powered, short range communication between devices where one of the communicating devices is battery powered.

The glucose meter 302 can be any glucose test system including a glucose test strip, a transducing sensor configured to determine the blood glucose level of a patient based on the sample on the test strip, and a communication device for sending the test result of the glucose test to a separate computing system, such as the monitoring system 304 or a remote system 310.

The monitoring system 304 can be any generalized computing system, but in particular example embodiments includes a portable, modular multiuser wellness parameter transducing system, such as the Cardiocom® Commander device.

Preferably, the monitoring systems 304 are all operatively connected to a remote system 310, such as over a network 312. The remote system 310 can be any of a number of generalized computing systems, such as the one disclosed above in conjunction with FIG. 2.

The remote system 310 contains a database 314. The database 314 stores patient data received from the monitoring systems 310. The patient data generally includes a patient identifier associated with test results from blood glucose tests; however, a wide variety of additional information can be stored in the database 314 as well. For example, the patient's medical history, current therapy regimen, family history, and/or socioeconomic health factors can be incorporated into the database 314. In certain specific embodiments, a patient's historical test results are stored.

In further embodiments, a device identifier can be stored in the database 314. The device identifier can be a unique identifier of the glucose meter 302, the monitoring system 310, or other system from which data is collected in the database 314.

A plurality of workstations 316 are also connected to the network 312. The network 312 can be any of a number of industry standard or proprietary data transmission networks, including local area networks (LAN), wide area networks (WAN), or internet or other web-based networks. The network can for example be packet or signal based, and can use any of a number of transmission protocols such as TCP/IP or other similar systems.

The workstations can any type of generalized computing system such as the one disclosed above in conjunction with FIG. 2. The workstations 316 are configured to communicatively connect to the remote system 310 over the network 312 in order to access the contents of the database 314. The workstations 316 may be used by either a patient or health care providers attending to that patient in order to access records associated with that patient.

For example, a patient may be authorized to access his or her historical records stored in the database 314. The patient can log onto a workstation 316 and access his or her health records via a webpage generated and personalized for that patient. The webpage could include personal health tips or other information relevant to the health concerns the patient may be experiencing. The webpage can be generated by, for example, the remote system 310 or another computing system connected to the network 312.

Alternately, the health care provider could be authorized to access the historical records of one or more patients stored in the database 314. The health care provider could inspect the daily records of the patients 314, or could choose to only inspect records for which an alert is generated consistent with the present disclosure. The health care provider could access these records via a client side application or web portal, and could use the data (test results, patient history, etc.) to contact the patient and intervene in the patient's medical treatment if necessary.

In various possible embodiments of the present disclosure, the remote system 310 is configured as a web server. In such an embodiment, the remote system 310 receives data requests from the workstations 316 or the monitoring systems 304, and provides browser-compatible data responsive to the requests. The monitoring systems 304 and/or the workstations 316 are configured to display the data, for example in a web browser such as Microsoft Internet Explorer, Netscape Navigator, Mozilla Firefox, Opera, or other similar browser software. Alternately, the remote system 310 can be configured to generate an alternate file type or data structure recognizable by the monitoring systems 304 and the workstations 316.

It is preferred that all monitoring systems 304 use the same type of communication link so that any one of the monitoring systems can readily connect to a given glucose meter 302. In this way, so long as the glucose meter 302 is communicatively linked to any one of the monitoring systems 304, the glucose meter 302 can connect to a monitoring system 304 at any one of the multiple locations at which a monitoring system 304 can reside. In such a configuration, the glucose meter can provide a unique identifier of the patient, as described below in conjunction with FIG. 5. In additional embodiments, the patient will carry or possess a unique identifier that is used to interface with the monitoring system 304. The unique identifier can be used to associate the test results from the glucose meter 302 with the patient when the data is stored in the database 314.

The system 300 can be used to analyze the patient's blood glucose trend and historical data. If significant symptoms are reported, the system 300 alerts the health care provider via email, phone call, or other communication, who may provoke a change to the patient's medication, health regimen, or establish further communication with the patient such as placing a telephone call to the patient. The communication between the patient's location 308 and the remote system 310 may be one way or two way communication depending on the particular situation.

Specifically, the following tables show blood glucose ranges that are within a "safe" range and results that could indicate onset of/ or previously undetected diabetes, or uncontrolled diabetes. A series of test results (i.e. a series of days with high blood glucose, etc.) well above or into the diabetic range can indicate a need for tighter glucose monitoring, diet or insulin management changes, or additional medical attention. In such cases, the remote system 310 can generate an alert to the health care provider, who can follow up with the patient as necessary with a phone call or other intervention.

| **Table 1: Fasting Blood Glucose** | |
|---|---|
| From 70 to 99 mg/dL (39 to 5.5 mmol/L) | Normal glucose tolerance |
| From 100 to 125 mg/dL (5.6 to 6.9 mmol/L) | Impaired fasting glucose (pre-diabetes) |
| 126 mg/dL (7.0 mmol/L) and above on more than one testing occasion | Diabetes |

| **Table 2: Oral Glucose Tolerance Test (OGTT) [except pregnancy] (2 hours after a 75-gram glucose drink)** | |
|---|---|
| Less than 140 mg/dL (7.8 mmol/L) | Normal glucose tolerance |
| From 140 to 200 mg/dL (7.8 to 11.1 mmol/L) | Impaired glucose tolerance (pre-diabetes) |
| Over 200 mg/dL (11.1 mmol/L) on more than one testing occasion | Diabetes |

| **Table 3: Gestation Diabetes Screening: Glucose Challenge Test (1 hour after a 50-gram glucose drink)** | |
|---|---|
| Less than 140* mg/dL (7.8 mmol/L) | Normal glucose tolerance |
| 140* mg/dL (7.8 mmol/L) and over | Abnormal, needs OGTT (see below) |
| Some use a cutoff of >130 mg/dL (7.2 mmol/L) because that identifies 90% of women with gestational diabetes, compared to 80% identified using the threshold of >140 mg/dL (7.8 mmol/L). | |

| **Table 4 : Gestation Diabetes Diagnostic: OGTT(100-gram glucose drink)** | |
|---|---|
| Fasting* | 95 mg/dL (5.3 mmol/L) |
| 1 hour after glucose load* | 180 mg/dL (10.0 mmol/L) |
| 2 hours after glucose load* | 155 mg/dL (8.6mmol/L) |
| 3 hours after glucose load* ** | 140 mg/dL (7.8 mmol/L) |
| *If two or more values are above the criteria, gestational diabetes is diagnosed. | |
| ** A 75-gram glucose load may be used, although this method is not as well validated as the 100-gram OGTT; the 3-hour sample is not drawn if 75 grams is used. | |

Source: ttp://www.labtestsonline.org/understanding/analytes/glucose/test.html

Referring now to FIG. 4, a blood glucose monitoring system 400 is shown according to another possible embodiment of the present disclosure. In this embodiment, the system 400 includes glucose meters 402 operatively connected to a remote system 404 through a network 406.

The glucose meters 402 of this embodiment are configured to communicate directly across the network 406 without a relay by a monitoring system such as is shown in FIG. 3. For example, the glucose meters 402 can include a networking link such as a copper or fiberoptic connection, 802.11a/b/g wireless connection, or other standard or proprietary networking connection. Such an embodiment is particularly advantageous in situations where monitoring systems, as shown in FIG. 3, are not available, i.e. when a patient is traveling or otherwise away from a monitoring system for an extended period of time.

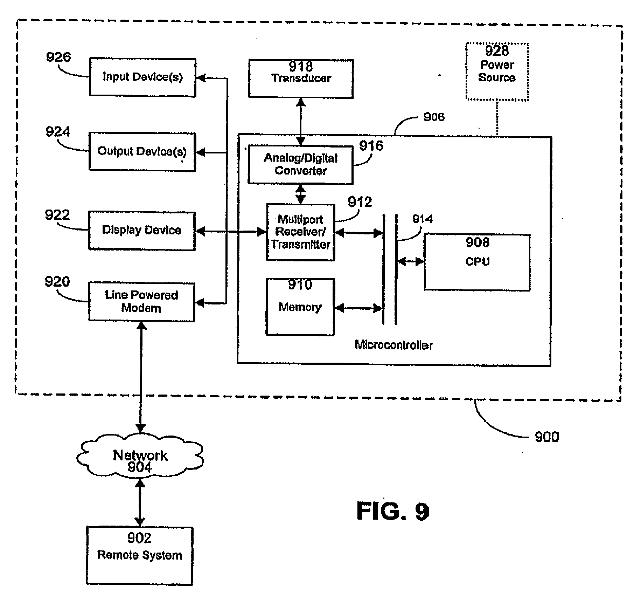

In particular embodiments, the glucose meter 402 can include or be locally connected to a line-powered modem 405, allowing the system to connect to the network 406 without the need to power a communications device. The system 400 can therefore incorporate a networking device without sacrificing battery life. Possible embodiments incorporating a line-powered modem 405 are shown in greater detail below in conjunction with FIGS. 9-10, 14.

Preferably, the remote system 406 is configured similar to the system 310 of FIG. 3. The remote system 406 stores patient data in a database 408, as described above. The data is available to patients or health care providers via browser or other document format when accessing the database 408 from the workstations 410.

Referring now to FIG. 5, a monitoring system 500 is shown according to a possible embodiment of the present disclosure. The monitoring system 500 forms an environment in which aspects of the present disclosure may be employed. The monitoring system 500 is configured to accept blood glucose test results from a glucose meter.

The embodiment of system 500 as shown incorporates a patient identification device 502. The patient identification device 502 is configured to determine if a person trying to use the system is one who is among a plurality of patients that are allowed or authorized to use the system 500. The device 502 selects one patient from among a plurality of patients that are allowed to use the system 500. By including such a patient identification device 502, any one system 500 can accept test results from multiple patients.

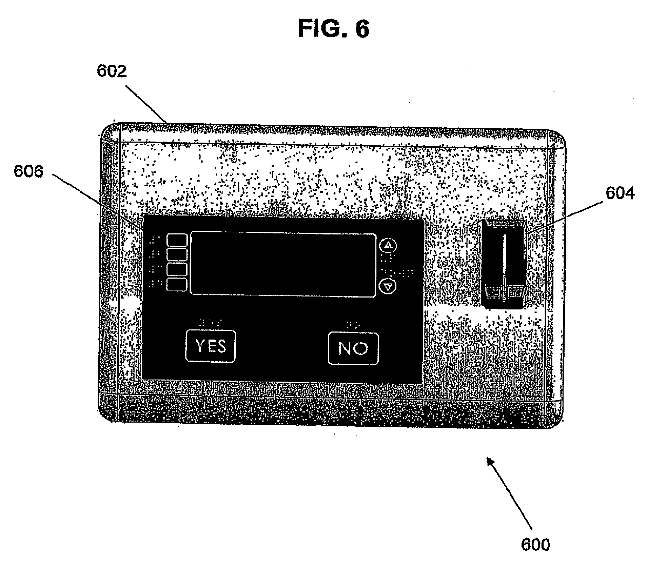

The patient identification device 502 can select the patient by interfacing with an identifier 504. The identifier 504 can be one or more of the identifiers that correspond to the patient identification device 502 resident in the system 500. In various embodiments, the identifier 504 can be a smart card or other card including a magnetic strip, wireless communication component, or bar code. In further embodiments, the identifier 508 can be an RPID tag, a biometric identifier unique to a patient, or an alphanumeric password system. Other suitable access means can also be used. The monitoring system 500 generally will include a patient identification device 502 that corresponds to the desired patient identifier 504, one embodiment of which is described below in conjunction with FIGS. 6-7.

The identifier 504 can include a memory. In embodiments where the identifier incorporates a memory, the patient identification device 502 includes an interface to the memory, allowing the system 500 to read or write data to the identifier.

In use, the system 500 measures one or more wellness parameters, for example blood glucose, glycosylated hemoglobin, weight, or blood pressure consistent with the disclosure herein. The system could also measure the weight of the patient. By detecting the identity of the patient, the blood glucose measurement can be associated with the identification of the patient, allowing multiple patients to use the same monitoring system 500 and associate test results with the correct patient and thereby placing those results in the correct record.

The patient identification device 502 can be any of a number of devices configured to interface with a selected patient identifier 504. In a preferred embodiment, the patient identification device 502 is a smart card reader, as shown below in conjunction with FIGS. 6-7. The smart card reader can be any type of card reader, from a magnetic strip reader, to a short range wireless transceiver, to a bar code reader. The patient identification device 502 can also be, for example, an RFID transceiver, a password authentication system, or a biometric sensor such as a fingerprint reader or voice recognition system. In one particular embodiment below, the patient identification device 502 is an ISO 7816 smart card reader incorporating a RS-232 interface chip manufactured by Microchip Technology, Inc. The needed firmware for controlling such a system can be incorporated in the memory 540 resident in the system 500.

A smart card is generally understood to be any pocket-sized card With embedded integrated circuits. Such cards can include memory and processing capabilities. Memory cards contain only non-volatile memory storage components, and perhaps some specific security logic. Microprocessor cards contain memory and microprocessor components. Smart cards are generally cards of credit card-like dimensions that are often tamper-resistant. Smart cards include contact (magnetic strip or interface) and contactless (generally RFID) smart cards.

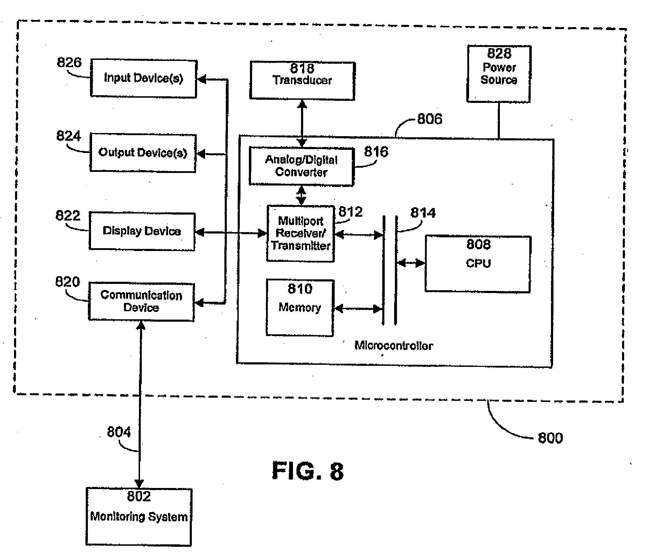

It is noted in the present disclosure that alternate patient identifiers 504 can be used as well, particularly in the case where the monitoring system 500 is absent from the overall system as shown in FIG. 4. For example, the glucose meters shown below in conjunction with FIG. 8-16 could include a unique identifier, such as a personal code or other unique identification such that the glucose meter can communicate the identification of the meter alongside any test results to a remote system. The glucose meters can also include a device identifier unique to the glucose meter. In this way, the overall system can associate the patient or device identification with stored test results in the database of the remote system of FIGS. 3-4.

Various alternate embodiments of the microprocessor system 500 can include the patient identification device 502. For example, the system 500 can include the patient identification device 502 in systems incorporating a wide variety of physiological parameter transducing devices, such as the glucose meter described below. Other physiological parameter that could be measured using similar systems and associated with a patient include weight, blood oxygen level, blood pressure, transthoracic impedance (examples of measured variables), or may be a value or score describing a patient's self-reported symptoms. Other physiological parameters can also be measured, tested, or communicated.

It is noted that for simplicity of design, a single type of patient identification device is used in conjunction with a single type of patient identifier in the embodiment described. However, it is recognized that additional types of patient identification devices can be used in conjunction with multiple patient identifiers in order to provide redundancy. This may be advantageous in situations where a patient loses an identification card, forgets a password, or otherwise is unable to use the primary mode of identification in the system 500.

As shown microprocessor system 524 includes a CPU 538, a memory 540, an optional input/output (I/O) controller 542 and a bus controller 544. It will be appreciated that the microprocessor system 524 is available in a wide variety of configurations and is based on CPU chips such as the Intel, Motorola or Microchip PIC family of microprocessors or microcontrollers.

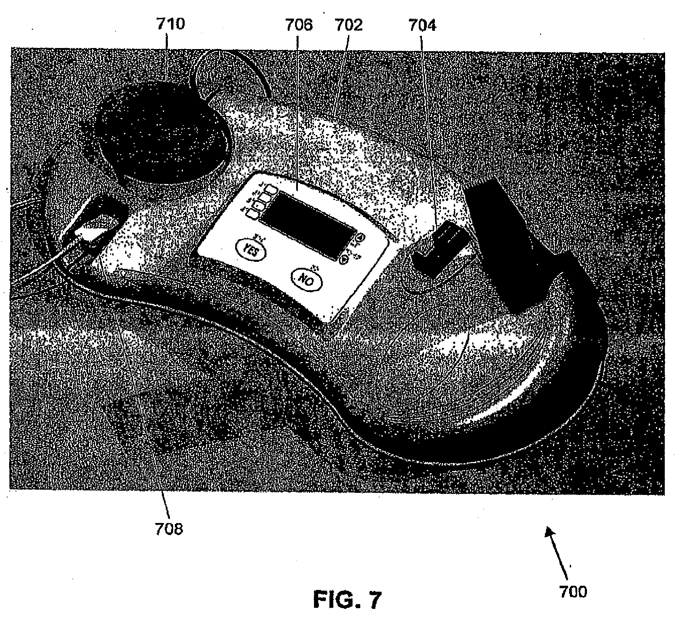

The microprocessor system 524 can be interfaced with a transducing device 518. The transducing device 518 can be any of a number of physiological parameter transducers. For example, the transducing device 518 could be a glucose meter 518. In further embodiments, the transducing device 518 could be a blood pressure cuff or pulse oximeter as described below in conjunction with FIG. 7. Additional embodiments of the transducing device 518 may include a glucose meter, spirometer, or other typical monitors. It is noted that the type of the transducing device 518 is not germane to the present disclosure.

It will be appreciated by those skilled in the art that the monitoring system 500 requires an electrical power source 519 to operate. As such, the monitoring system 500 can be powered by: ordinary household A/C line power, DC batteries or rechargeable batteries, or other power sources. The power source 519 provides electrical power to the housing for operating the electronic devices.

The housing 514 includes a microprocessor system 524, an electronic receiver/transmitter communication device 536, an input device 528 and an output device 530. The communication device 536 is operatively coupled to the microprocessor system 524 via the electronic bus 546, and to a remote computer 532 via a communication network 534 and a communication device 535. The communication network 534 can be any communication network such as a telephone network, wireless network, wide area network, or Internet. It will be appreciated that the communication device 536 can be a generally known wired or wireless communication device. For example, the device 536 can be any packet-based or wave-based wireless communication device operating using any of a number of transmission protocols, such as 802.11a/b/g, bluetooth, RF, cellular (CDMA or GSM) or other wireless configurations. The device can alternately or additionally incorporate a wired device, such as a modem or other wired internet connection.

It will be appreciated that output device(s) 530 may be interfaced with the microprocessor system 524. These output devices 530 can include a visual electronic display device 531 and/or a speech device 533. Electronic display devices 531 are well known in the art and are available in a variety of technologies such as vacuum fluorescent, liquid crystal or Light Emitting Diode (LED). The patient can read alphanumeric data as it scrolls on the electronic display device 531. Output devices 530 can include a synthetic speech output device 533 such as a Chipcorder manufactured by ISD (part No. 4003), electronic sound file playback system (WAV, MP3, etc.), or voice synthesizer. Still, other output devices 530 include pacemaker data input devices, drug infusion pumps, or transformer coupled transmitters.

It will be appreciated that input device(s) 528 may be interfaced with the microprocessor system 524. In one embodiment of the present disclosure an electronic keypad 529 is provided for the patient to enter responses into the monitoring system 500. Patient data entered through the electronic keypad 529 may be scrolled on the electronic display 531 or played back on the synthetic speech device 533.

Preferably, the microprocessor system 524 is operatively coupled to the communication device 536, the input device(s) 528 and the output device(s) 530.

Referring now to FIGS. 6-7, two possible physical structures of monitoring systems 600, 700 are shown. Preferably, these systems are small, portable devices that are configured to be placed in a wide variety of healthcare related and non-healthcare related locations in order to facilitate patient interaction and health history tracking on a large population without having to outfit each potential patient with such an apparatus. Specifically, the systems 600, 700 can be placed in a workplace to ensure regular monitoring, leading to potential early intervention regarding potential health issues of workers.

Referring now to FIG. 6, a physical structure of a monitoring system 600 is shown according to one possible embodiment. In the embodiment shown, the monitoring system 600 has a body 602 that incorporates a personal identification device 604 and a panel 606 incorporating input devices and output devices.

The personal identification device 604 can be any of a number of identification devices as described above in conjunction with FIG. 5. In the embodiment shown, the device 604 includes an ISO 7816 standard smart card reader interfaced to the circuitry as shown in FIG. 5 through a USB or RS-232 interface chip, such as are manufactured by Microchip Technologies, Inc.

The panel 606 can incorporate input and output devices as shown in FIG. 5 and described above in conjunction with FIGS. 4-6.

In use, a patient would activate the monitoring system 600 by sliding a smart card into the personal identification device 604 shown. The system 600 would then determine if the patient is a recognized user by either accessing internal memory, data stored on the smart card, or a remote memory connected to the system 600 over a communication network.

In the embodiment shown, the monitoring system 600 can incorporate a physiological parameter transducing device (not shown), or can alternately include linkages to such devices.

Referring now to FIG. 7, a possible structural embodiment of the multiuser wellness parameter monitoring system 700 is shown. In this embodiment, the system 700 can be used as a "kiosk" placed in a variety of locations at which persons may congregate and either require or be interested in a heath status update. The system 700 has a body 702 that incorporates a personal identification device 704 and a panel 706 incorporating input devices and output devices. In the embodiment shown, the body 702 is generally rounded and includes molded forms that can hold physiological parameter transducing devices, such as a pulse oximeter 708 and a blood pressure cuff 710.

The pulse oximeter 708 can be any of a number of widely available oximeter products on the market. Such pulse oximeters 708 can measure the patient's heart rate and/or blood oxygen level. The blood pressure cuff 710 can be any of a number of blood pressure cuffs widely available as well. Of course, any number of additional physiological parameter transducing devices could be integrated with the apparatus 700 consistent with the present disclosure.

Referring now to FIG. 8, a block diagram of a glucose meter 800 is shown according to a possible embodiment. In the embodiment shown, the glucose meter 800 is connected to a monitoring system 802 via a communication link 804. The communication link 804 can be any of a number of wired or wireless communication links such as Infrared, Bluetooth, Universal Serial Bus, or RS-232. Preferably, the glucose meter 800 includes a microcontroller system 806 having a microprocessor 808, a memory 810, and a receiver/transmitter 812 linked by a data bus 814.

The microprocessor 808 can be any of a number of embedded low power processors such as those made by Intel Corporation, Transmeta Corporation, Advanced Micro Devices, International Business Machines, Freescale Semiconductor, Microchip PIC or other suitable devices. The data bus 814 to which the microprocessor 808 is linked is configured to provide a data interface between the microprocessor 808, memory 810, and receiver transmitter 812.

The memory 810 contains computer-readable instructions for computing a result of a blood glucose test based on data received by the microprocessor 808 through the receiver/transmitter 812. The memory 810 also stores past results of blood glucose tests to show trends in blood glucose readings to the patient.

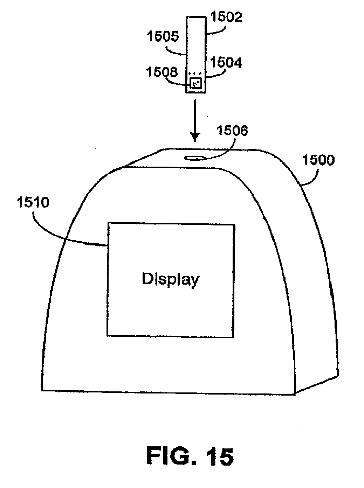

The receiver/transmitter 812 is operatively connected to an analog/digital converter 816. The analog/digital converter 816 is interfaced with a transducer 818. In preferred embodiments, the transducer 818 converts a blood glucose level to an electrical signal, which in turn is converted into a digital signal by the analog/digital converter 816. The transducer can interact with a test strip (for example seen in FIGS. 15-16) to read a glucose level in a blood sample on the test strip. Such blood glucose testing is important for patients with diabetes mellitus. Since approximately 1980, a primary goal of the management of type 1 diabetes has been the achievement of closer-to-normal levels of glucose in the blood for as much of the time as possible, guided by blood glucose tests conducted several times a day. This has greatly increased the time spent in the daily care of this disease but has also reduced rates of long-term complications and improved the management of short-term, potentially life-threatening complications.

In alternate embodiments, the transducer 818 measures the glycosylated hemoglobin of a patient. Measurement of glycosylated hemoglobin or hemoglobin A1c (HgbA1c) is a valuable tool in the monitoring of diabetic patients, and those patient's with insulin resistance. Glycosylation is the nonenzymatic addition of a sugar residue to amino groups of proteins. Formation of glycosylated hemoglobin is essentially irreversible and the blood level depends on both the lifespan of the red blood cell (approximately 120 days) and the blood glucose concentration. Because the rate of formation of glycosylated hemoglobin is directly proportional to the blood glucose concentration, the HgbA1c represents the integrated values for the glucose concentration over the preceding 8-12 weeks. The measured value of glycosylated hemoglobin is weighted to the most recent glucose values. The most recent 30 days represent roughly 50% of the glycosylated hemoglobin level, while the preceding 60 days and then 90 days each representing a quarter of the glycosylated hemoglobin level, respectively. Glycosylated hemoglobin measurements have the advantage that they are not subject to the fluctuations that are seen with daily glucose monitoring.

The American Diabetes Association (ADA) recommends glycosylated hemoglobin as the best test to find out if a patient's blood sugar is under control over time. Further, studies by the Diabetes Control and Complications Trial (DCCT) and the United Kingdom Prospective Diabetes Study (UKPDS) showed that the lower the test result number, the greater the chances to slow or present the development of serious eye, kidney and nerve disease. The studies also showed that any improvement in glycosylated hemoglobin levels can potentially reduce complications.

The ADA recommends that action be taken when glycosylated hemoglobin results are over 8%, and considers the diabetes to be under control when the test result is 7% or less. The following table shows the relationship between glycosylated hemoglobin and blood glucose levels.

| **HbA1c %** | **Mean Blood Glucose (mg/dL)** | **Average Plasma Glucose (mg/dL)** | **Interpretation** |
|---|---|---|---|
| 4 | 61 | 65 | Non-Diabetic Range |
| 5 | 92 | 100 | |
| 6 | 124 | 135 | |
| 7 | 156 | 170 | Target for Diabetes in Control |
| 8 | 188 | 205 | Action Suggested according to ADA guidelines |
| 9 | 219 | 240 | |
| 10 | 251 | 275 | |
| 11 | 283 | 310 | |
| 12 | 314 | 345 | |

Source: http://web.missouri.edu/∼diabetes/ngsp/ghbmbg/ghbmbg.htm; Diabetes Care 2004;27 (Suppl. 1):S91 - S93.

Referring still to FIG. 8, the glucose meter 800 also includes a communication device 820, display device 822, output devices 824, and input devices 826 connected to the receiver/transmitter 812. The communication device 820 is a device configured to send and receive data according to a format recognizable by the remote system 804. In various embodiments, the communication device 820 is a bluetooth receiver/transmitter, an infrared receiver/transmitter, a USB controller, a serial controller, or other wired or wireless data controller. In preferred embodiments, the communication device 820 is a low-powered communication receiver/transmitter powered by a power source 828 that can be used in devices in which battery life is important. In further embodiments, the communication device can be powered by a signal from the communication link 804.

The display device 822 can be any type of generally low powered displays capable of producing a representation of the test result computed in the glucose meter 800 based on the sample read by the transducer 818 when interfaced, for example, with a glucose test strip. In various embodiments, the display device 822 is an LED display, a liquid crystal display, or other similar display types.

The output devices 824 can be any of a number of additional display, audio, or other output devices included in the glucose meter 800 and configured to output data stored in the glucose meter. In further embodiments, the display device 822 is the only output device.

The input devices 826 can be any number of devices configured to allow a patient using the glucose meter 800 to select and provide input commands to the meter. The input devices 826 can include pushbuttons, a touch screen display, voice recognition, a scroll wheel or joystick, or any other input device. The input devices 826 allow the user to provide commands to the glucose meter, for example, to request a display of historical blood glucose test results stored in the memory 810; to start a blood glucose test upon insertion of a test strip; or to turn the meter 800 on or off.

In the embodiment shown, the glucose meter 800 is powered by a power source 828 included within the meter 800. For example, the power source 828 can be a single use or rechargeable battery. In further embodiments, the power source 828 can be an AC or DC outlet for plugging into a wall outlet, base station, or car charger. ,

Referring now to FIG. 9, a block diagram of a glucose meter 900 is shown according to a possible embodiment. In the embodiment shown, the glucose meter 900 is directly connected to a remote system 902 via a network 904. The remote system can be any suitable remote computing system, such as the systems shown in FIGS. 2-4.

The glucose meter 900 includes the same basic components as the meter 800 in FIG. 8. However, in certain embodiments of the glucose meter 900, a power source 928 is unnecessary. In such embodiments, the meter 900 receives power from an external source, such as through an RJ-11 plug and routed from a line-powered modem 920 as discussed below.

In the embodiment shown, the meter 900 includes a line-powered modem 920. The line-powered modem 920 can be a modem of a wide variety of speeds/protocols, such as v.92 or other similar modem communications protocols. The line-powered modem 920 generally connects to an RJ-11 telephone jack, and receives signals from the network on that jack connection. It is understood that an intermediate modem pool (not shown) can provide the Internet-to-analog conversion required to convert the packet-based TCP/IP signals commonly found in internet communications to the analog signals used in telephony/modem communications.

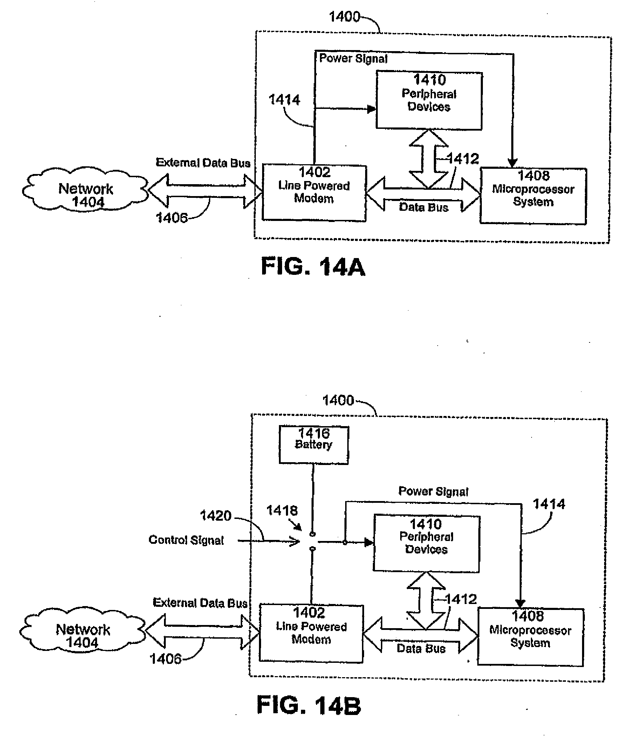

Line-powered modems are particularly useful in applications where an external power source is not available. The line-powered modem 920 is able to use received analog signals to power the internal circuitry of the modem as well as a certain amount of additional circuitry, dependent upon the power demands of the circuitry as compared to the power receivable on signals by the modem through the RJ-11 port. Specific power distribution arrangements are shown and described in FIGS. 14A-B.

In one possible embodiment, the line-powered modem 920 may include a wake-on-ring feature wherein the remote system 902 could send a signal to the glucose meter 900. The line-powered modem 920 could receive the signal and recognize the signal as an indication that the system should be powered. Following any necessary initialization steps, the glucose meter 900 could communicate with the remote system 902, for example sending glucose test measurements recently measured by the meter 900. In further embodiments, the line-powered modem 920 is used for communications sessions in which the glucose meter 900 instantiates the communication session with the remote system 902.

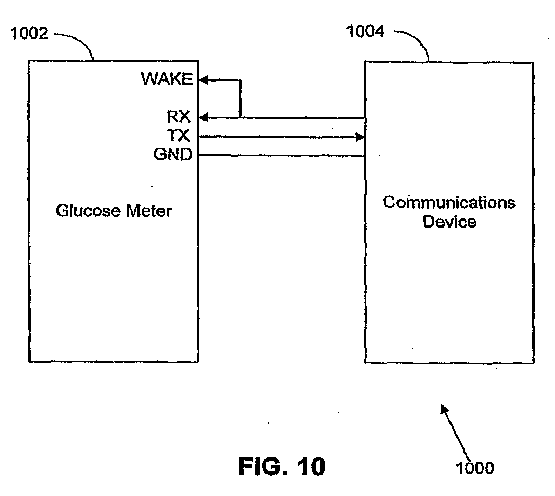

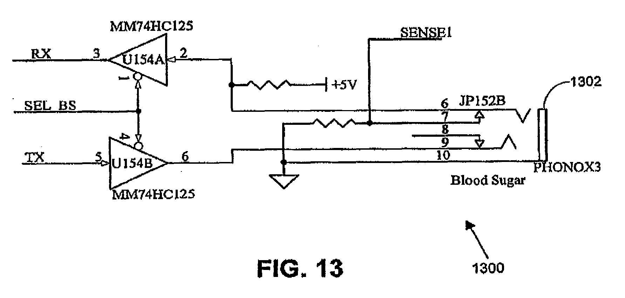

Referring now to FIG. 10, a connection diagram of a portion of a blood glucose monitoring system 1000 is shown. In the system 1000, a glucose meter 1002 does not include a communications device other than a standard receiver/transmitter arrangement, included with the blood glucose meter circuitry of FIG. 13. The system 1000 includes both the glucose meter 1002 and a communications device 1004. Preferably, the communications device 1004 is a line-powered communications device, resides external to the glucose meter, and is connected via transmit, receive, ground, and wake signals. The communications device 1004 can be a line-powered modem, and can be used to distribute power as shown below in conjunction with FIG. 14.

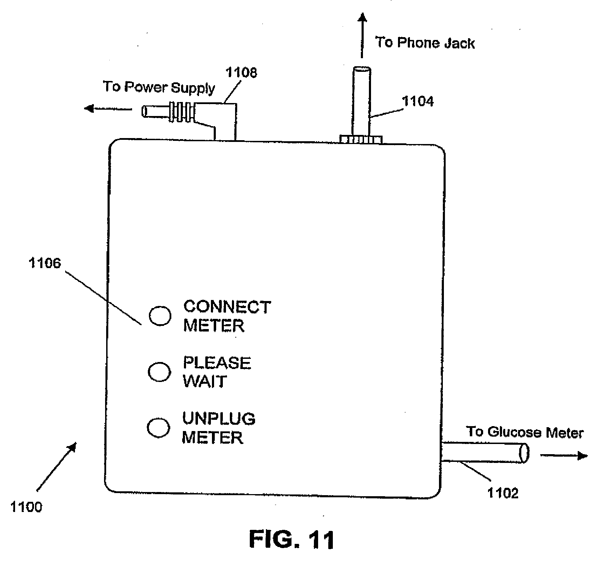

Referring now to FIG. 11, a schematic view of a communications device 1100 is shown according to a possible embodiment of the present disclosure. The communications device 1100 is configured for local use in conjunction with a glucose meter, and can communicate test results from the glucose meter to the remote system or monitoring system as shown above in FIGS. 3-4.

The communications device 1100 has a communicative connection 1102 to a glucose meter. The communicative connection 1102 is a unidirectional or bidirectional link capable of allowing the communications device to access and download data such as glucose meter modes or test results computed by the glucose meter. The communicative connection 1102 can be a standard or proprietary connection. In a possible embodiment, the connection is accomplished via a stereo mini jack interfaceable to a glucose meter. Of course, additional connective configurations are possible.

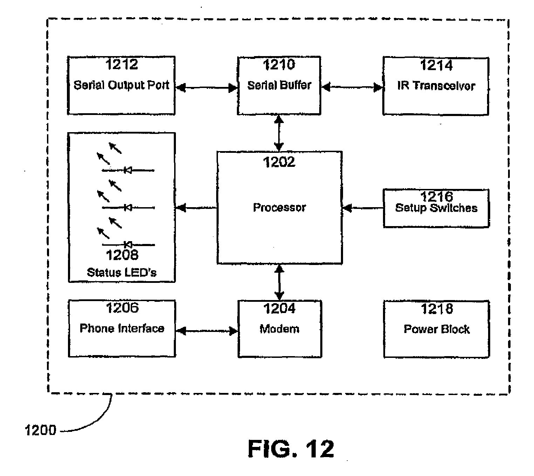

The communications device 1100 further includes a network connection 1104. The network connection shown is a phone line connection that connects via an RJ-11 jack installed in the communications device 1100. The RJ-11 jack can in turn route communications signals to and from a modem internal to the communications device 1100, as shown for example in FIG. 12. Alternately, the communications device 1100 can include alternate communications devices, such as a 10/100 ethernet PHY transceiver, a wireless device such as by 802.11 a/b/g or WiMAX, or other communications devices.

The communications device 1100 includes an indicator panel 1106. In the embodiment shown, the indicator panel includes a series of three indicators, such as light-emitting diodes. The light emitting diodes can be a number of different colors so as to be readily distinguishable, such as green, yellow, and red, respectively. Each diode can be associated with a message to be communicated to a user of the communications device 1100 (and associated glucose meter) that are printed on the face of the device near the indicator panel. In one embodiment of communications device 1100, the messages "CONNECT METER", "PLEASE WAIT", and "UNPLUG METER" are each associated with a separate diode that can be activated to indicate to the user the current status of the communications device 1100. In a possible configuration of the communications device 1100, the "CONNECT METER" message is associated with a yellow LED, the "PLEASE WALT" message is associated with a red LED, and the "UNPLUG METER" message is associated with a green LED.

The communications device 1100 can also include a power input 1108. The power input 1108 can be operable in conjunction with an alternating current or direct current power supply, and preferably provides a direct current source to the communications device 1100 at a predetermined voltage.

In use, the communications device 1100 can be connected to or disconnected from a glucose meter. When the glucose meter and the communications device 1100 are not connected and the communications device 1100 is receiving power via the power input 1108, the communications device 1100 can be configured to illuminate a LED corresponding to the "CONNECT METER" message. The communications device 1100 can maintain illumination of that LED until the device 1100 senses that a connection has been established between it and a glucose meter.

When the communications device 1100 senses a connection to a glucose meter, it can attempt to access data stored in a memory resident within the glucose meter. The data can include user information, glucose meter information, and glucose test results, and can be accessed consistent with the methods and systems described below in conjunction with FTGS. 17-28. While the communications device 1100 is accessing data stored within the glucose meter, it is preferable that the devices remain connected. The communications device can therefore deactivate the LED associated with the "CONNECT METER" message and can activate the LED associated with the "PLEASE WAIT" message.

When the communications device 1100 has completed its data acquisition from the glucose meter, the LED associated with the "PLEASE WAIT" message can be deactivated and the LED associated with the "UNPLUG METER" message can be activated. This could indicate to the user that communication between the devices has completed and the glucose meter can safely be disconnected.

Referring now to FIG. 12, a block diagram of a communications device 1200 is shown according to a possible embodiment of the present disclosure. The communications device 1200 can be, for example, the functional components of the communications device 1100 of FIG. 11.

The communications device 1200 includes a processor 1202. The processor 1202 can be any of a number of processors described herein, and can be configured to control the operation of the system 1200 as a whole. The processor 1202 controls data handling by the communications device 1200 by coordinating the surrounding modules described below.

The communications device 1200 further includes a modem 1204. The modem 1204 operates at one or more BAUD rates and operable on one or more protocols (v.90, v.92, etc.), and is configured to communicatively connect to a network, such as the one shown above in FIGS. 3-4. The modem 1204 can be a line-powered modem or can accept power from a separate power supply as shown.

The modem 1204 is in turn connected to a phone interface 1206. The phone interface RJ-11 is generally an RJ-11 jack configured to accept a complementary plug to establish a communicative connection. Other jack or connection interfaces are possible as well.

The processor 1202 is operatively connected to a display panel 1208, shown as a series of light emitting diodes that indicate the status of the device 1200. The display panel 1208 preferably indicates the status of the device to a user so that the user can easily determine the current operation of the device 1200 and react accordingly. For example, the display panel 1208 can be the series of LEDs shown in FIG. 11, which indicate when intervention from a user of the device is appropriate by illuminating an LED associated with a message printed on the face of the communications device 1200.

The processor 1202 is further coupled to a serial buffer 1210. The serial buffer 1210 is a bidirectional, multiport buffer configured to facilitate communication between the processor 1202 and one or more external devices. In the embodiment shown, the serial buffer 1210 includes links to a serial output port 1212 and an infrared transceiver 1214. The serial output port 1212 allows for a serial communication connection to be made between the communications device 1200 and an external device, such as a glucose meter. The infrared transceiver 1214 provides an alternative communicative connection between the communication device 1200 and a nearby component such as a glucose meter configured with an IR communications system.

The processor 1202 is additionally connected to one or more setup switches 1216. The setup switches 1216 can control any of a number of aspects of the communications device 1200, such as to coordinate communication via the serial output port 1212, the modem 1208, or the infrared transceiver 1214. The setup switches 1216 may or may not be accessible external to the communications device 1200. For example, the setup switches 1216 can be user control switches configured to allow a patient to operate the communications device 1200 in accordance with a specific glucose meter. In an alternative embodiment, the setup switches 1216 are DIP switches set by the manufacturer or deployer of the communications device 1200 so as to coordinate the communications device 1200 to communicate with a specific remote system or monitoring system, such as are shown above in conjunction with FIGS. 2-7.

The communications device 1200 can further include a power block 1218 configured to distribute a power signal throughout the device 1200. The power block is present in embodiments of the communications device 1200 that do not include a line-powered communications device as described herein, and may be optional where such a device is included in the communications device 1200. Preferably, the power block 1218 provides a constant DC power source to the communications system at a specified voltage. In one embodiment of the present disclosure, the predetermined voltage can be selectable using the setup switches 1216 described above.

Referring now to FIG. 13, internal circuitry for a glucose meter 1300 is shown. The glucose meter 1300 can include integrated circuitry configured to provide asynchronous receipt and transmission of data in the glucose meter 1300. A glucose strip 1302 is inserted in the glucose meter 1300 and is configured to operate in conjunction with the internal circuitry of the glucose meter 1300 to provide a test result. The test result can be, for example, a test result representative of the glucose concentration in the patient's plasma component of their blood.

The glucose meter 1300 can be used in conjunction with a variety of communication configurations, such as a separate communications device, line-powered or otherwise, as shown above in FIGS. 10-12, or can incorporate a line-powered modem as in FIG. 14. Additional communicative configurations incorporated into glucose meter 1300 can be implemented.

Referring now to FIGS. 14A-14B, a glucose meter 1400 is shown according to a particular embodiment of the present disclosure. FIG. 14A shows a configuration of a glucose meter 1400 powered by a line-powered modem 1402. The line-powered modem 1402 is connected to a network 1404 via an external data bus 1406. The line-powered modem 1402 is interfaced with a microcontroller system 1408 and peripheral devices 1410 via both a data bus 1412 and a power signal 1414. The line-powered modem 1402 receives a signal on the external data bus 1406, and converts that signal to both a power signal 1414 and a data signal to be placed on the data bus 1412. Both the power signal 1414 and the data signal are transmitted from the line-powered modem 1402 throughout the glucose meter 1400.

In such an embodiments, the line-powered modem 1402 provides the power connections for the internal circuitry of the glucose meter 1400. Although a battery or other power source may be connected to such a system, there is no absolute need for a power source.

FIG. 14B shows a configuration of a glucose meter 1400 selectively powered by a line-powered modem 1402. The line-powered modem 1402 is connected to a network 1404 via an external data bus 1406. The line-powered modem 1402 is interfaced with a microcontroller system 1408 and peripheral devices 1410 via both a data bus 1412 and a power signal 1414. The line-powered modem 1402 receives a signal on the external data bus 1406, and converts that signal to both a power signal 1414 and a data signal to be placed on the data bus 1412. Both the power signal 1414 and the data signal are transmitted from the line-powered modem 1402 throughout the glucose meter 1400.

In the embodiment shown in FIG. 14B, the glucose meter 1400 also includes a battery 1416. Preferably, the battery 1416 is electrically connected to the power signal at a switch 1418. The switch 1418 controls whether the battery 1416 or the line-powered modem 1402 provides power to the microcontroller system 1408 and peripheral devices 1410 in the meter 1400.

A control signal 1420 operates to selectably switch the power source between connecting the line-powered modem 1402 and the battery 1416. The control signal 1420 can be based on, for example, the remaining capacity of the battery 1416, the strength of the signal received by the line-powered modem 1402 on the external data bus 1406, or other similar factors. Alternately, the control signal 1420 can be controlled by a user-activated switch, a signal from another portion of the device, or a signal from another device altogether.

Referring now to FIG. 15, a glucose meter 1500 is shown according to a possible embodiment. The glucose meter 1500 is configured to accept a test strip 1502. The test strip 1502 has an insertion portion 1504 and an exposed portion 1505. The insertion portion is placed into an opening 1506 in the glucose meter 1500. Preferably, the insertion portion 1504 includes a calibration code, shown as calibration identifier 1508, printed along the length of the test strip 1502. When the test strip 1502 is inserted into the opening 1506, the glucose meter 1500 reads the calibration identifier 1508.

In a possible embodiment, the calibration identifier 1508 is a bar code, and can be read, for example, with an infrared bar code reader. The bar code represents a code that is used to calibrate the glucose meter 1500 with respect to the particular properties of the test strip 1502.

In a further possible embodiment, the calibration identifier 1508 is an integrated circuit or other miniaturized memory device embedded in the test strip, and the test strip has leads that are electrically connected to the internal circuitry of the glucose meter 1500, allowing the glucose meter 1500 to read the memory embedded in calibration identifier 1508 and correspondingly calibrate the meter 1500. In such an embodiment, it is understood that the integrated circuit or miniaturized memory device itself need not be included on the insertion portion 1504; rather, an interface to the integrated circuit will be included on the insertion portion so as to interface with the glucose meter 1500.

Glucose meters, such as glucose meter 1500 can determine the blood glucose level of a patient by comparing a measured voltage, resistance, current, or other circuit value sensed in the test strip with known quantities. For example, the glucose meter 1500 can use a look-up table stored in memory to determine the accurate blood glucose concentration. The glucose meter 1500 could alternately calculate the blood glucose concentration.

Generally, before a patient uses a glucose meter 1500, that patient needs to calibrate the meter to the test strips 1502. This calibration must at least be done every time a new container of test strips is opened and before the first strip is used. This is because each batch of test strips, and potentially each test strip within a given batch, has varying characteristics that can change the performance of the strip. (i.e. there is a proportional difference in glucose detected based on the amount of hexokinase or other chemical on the strip). Some meters require that the patient push a button until the number that appears on the display corresponds to the number located on the test strip container. Other meters use strips that come with an encoded key or strip that allow patients to calibrate the meter by inserting the encoded key or strip into a slot in the meter. By providing a calibration identifier 1508 on each test strip 1502, accurate and reliable calibration is achieved automatically upon insertion of each test strip, eliminating the need for a separate calibration strip, a calibration chip, or manual code entry by a patient.

Of course, other types of calibration code systems than bar codes or integrated circuits could be used, including embedded resistance in the test strip corresponding to a calibration value, or other suitable techniques. It is understood that the description of the bar code and reader or integrated circuit and electrical leads herein in conjunction with the calibration identifier 1508 is not meant to limit the calibration technique, but is instead intended to encompass similar solutions for which calibration is an automatic result of inserting a test strip.

The glucose meter 1500 further includes a display 1510, such as a digital display. The display 1510 presents to the patient their test results once a sample is read by the meter 1500. The display 1510 can also present a variety of messages to the patient related to the insertion of a test strip 1502 and calibration of the meter 1500. For example, when the glucose meter 1500 is originally turned on, the meter may indicate that a test strip 1502 should be inserted. Once a test strip 1502 is inserted, a message can be presented to the patient that the calibration is in progress, or is completed, and that the glucose meter 1500 is ready to conduct a blood glucose test.

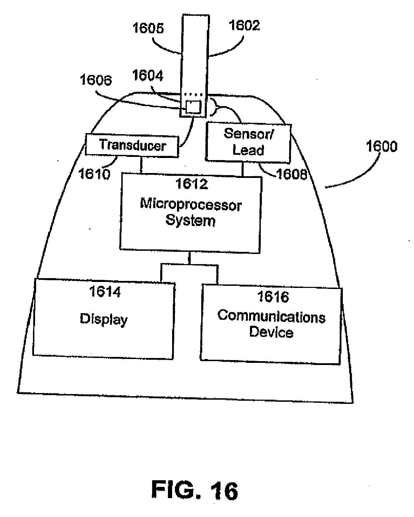

Referring now to FIG. 16, a block diagram of internal circuitry of a glucose meter 1600 is shown according to a possible embodiment of the present disclosure. In the embodiment shown, a test strip 1602 includes an insertion portion 1604 and an external portion 1605. The test strip 1602 can be inserted into the glucose meter 1600 such that the insertion portion 1604 resides within the meter 1600. A calibration identifier 1606 located on the insertion portion 1604 is interfaced with a calibration identifier access device, shown as sensor 1608.

The test strip 1602 is also interfaced with a transducer 1610, which detects the level of glucose in the blood sample on the test strip and converts that reading to an electrical signal representative of such a sample.

Both the transducer 1610 and the sensor 1608 are interfaced with a microcontroller system 1612. The microcontroller system can be, for example, either of the systems shown above in conjunction with FIGS. 8-9. Hence, when the microcontroller system 1612 receives the signal from the sensor 1608, the system 1612 can use the resultant signal to self-calibrate and produce accurate results based on the electrical signal produced by the transducer 1610 as read from the test strip 1602.

The microcontroller system 1612 is operatively connected to a display 1614 and a communications device 1616. The display 1614 can be any type of liquid crystal, diode, or other display capable of low power production of a signal for communication to a patient representative of the patient's blood glucose levels, i.e. test results. The communications device 1616 can be of any communications devices configured for long or short distance communication of the test results to either a monitoring system or a remote system, such as those described above in FIGS. 2-7.

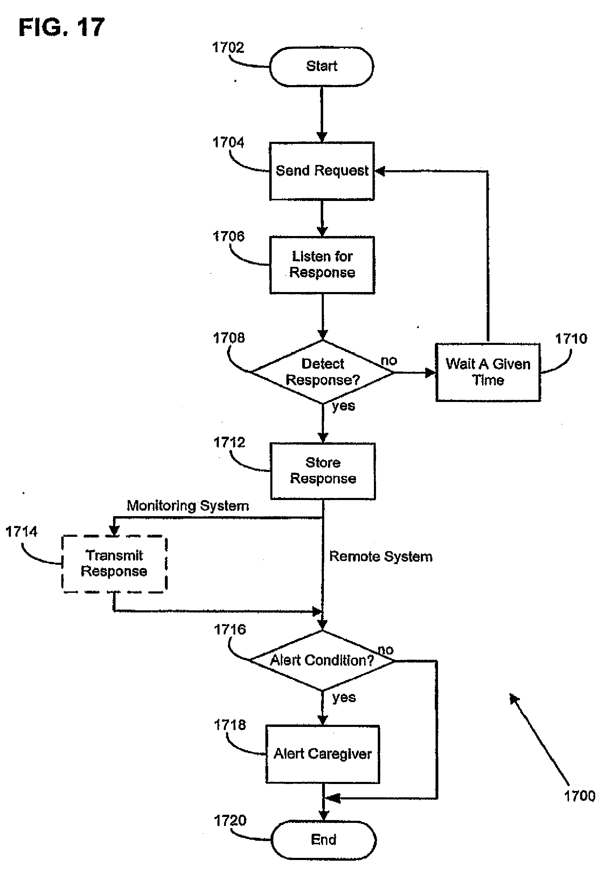

Referring now to FIG. 17, a flowchart of systems and methods for blood glucose monitoring is shown according to a possible embodiment of the present disclosure. The system 1700 as shown can be executed by either the monitoring system or remote system described above. Additionally, the system 1700 can be executed by a workstation affiliated with one or both of the remote or monitoring systems.

The system 1700 is instantiate by a start operation 1702, Operational flow proceeds to a request module 1704. The request module 1704 sends a request over a network or other communication link to a glucose meter, such as the glucose meters shown above in FIGS. 8-14. The request module 1704 is programmed to send such a request at a predetermined time. For example, the request module 1704 may be programmed to send such a request once or twice a day in order to receive updated glucose test results from tests performed by the glucose meter since the last request was sent.

A listen module 1706 is configured to wait for a response from any glucose meter within range of the system 1700. For example, the listen module may listen for one to five minutes to allow a glucose meter to respond to the request. The glucose meter responds in a manner recognized by the system 1700. For example, if the system sends a wireless broadcast request in the request module 1704, the listen module 1706 will listen for an analogous response.

A detection operation 1708 determines if a response by a glucose meter has been received by the listen module 1706. If the detection operation 1708 determines that a response is detected, operational flow branches "yes" to a store module 1712. If the detection operation 1508 determines that response is not detected, operational flow branches "no to a wait module 1710. The wait module 1710 holds the system for a given time in a "wait state". The given time can be the same as or less than the predetermined time between requests made by the request module 1704 as described above. For example, the wait module 1710 may wait an hour before passing operational flow to the request module. Or, the wait module 1710 may wait for the entire length of the predetermined time between requests. Once the wait state is completed, operational flow proceeds back to the request module 1744 for a repeated request of a glucose meter and repeated listening for a response, and operational flow proceeds as described above.

In this way, the system 1700 can send requests and listen for responses at a given frequency based on the time required for the request module 1704, the listen module 1706, the detect module 1708, and the wait module 1710 to execute. The given frequency may be reprogrammable based on adjustment of the time set in the wait module 1710.

The store module 1712 stores the test result associated with the patient data in a memory. In embodiments performed on the monitoring system, the store module stores the test result in a system memory alongside a patient identification as determined by interfacing with a patient identifier. In embodiments performed on a remote system, the store module 1712 stores the test result in a database such that the test result is accessible to a patient or health care provider at a remote workstation or monitoring system, such as is shown above in FIGS. 3-7.

After the test result is stored, the actual operational flow of the system 1700 depends upon the component in which the system 1700 operates. In the case of a system 1700 operating in a monitoring system such as is described above in conjunction with FIGS. 3-7, operational flow can optionally proceed to a transmit module 1714. The transmit module 1714 is generally performed in embodiments of the system 1700 resident upon a monitoring system such as the one shown above in FIGS. 3-7. In such embodiments, the transmit module 1714 transmits the test results to the remote system for long-term storage and requests by a patient or health care provider using a monitoring system or workstation. Following the transmit module, operational flow proceeds to an alert determination module 1716, below.

In the case of a system 1700 operating in a remote system such as is described above in FIGS. 2-4, there is limited need for a transmit operation 1714 because the computing system that generates alerts, such as to a health care provider or other caregiver (as described below), has the relevant data. In such a case, operational flow can proceed directly to an alert determination operation 1716. The given time can be the same as or less than the predetermined time between requests made by the request module 1704 as described above, or some other suitable time period.

The alert determination operation 1716 accesses data, such as the last test result received by the remote system or historical test result data. Based on the criteria previously described, the alert determination operation 1716 determines whether sending an alert to the health care provider would be appropriate.

If the alert determination operation 1716 determines that an alert is appropriate, operational flow branches "yes" to an alert generation module 1718. The alert generation module 1718 sends an alert notification to a caregiver of the patient, for example a health care provider at a workstation shown in FIGS. 3-4. The health care provider can review the patient record and determine what additional action would be appropriate given the specific reasons the alert was generated. For example, the health care provider may determine that the patient needs to change their diet, insulin, or oral agent regimen

The system terminates with an end module 1720, Referring back to the alert determination operation 1716, if the alert determination operation 1716 determines that an alert is not appropriate, operational flow branches "no" to the end module 1720, where operational flow terminates.

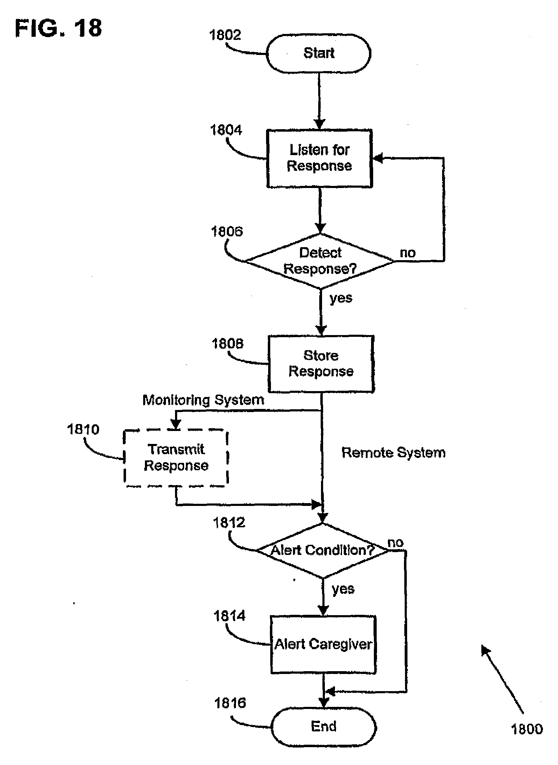

Referring now to FIG. 18, a flowchart of systems and methods for blood glucose monitoring is shown according to a possible embodiment of the present disclosure. The system 1800, as shown, can be executed by either the monitoring system or the remote system described above in FIGS. 2-7. Additionally, the system 1800 can be executed by a workstation affiliated with one or both of the remote or monitoring systems.

The system 1800 is instantiated by a start module 1802. Following the start module 1802, operational flow proceeds to a listen module 1804. The listen module 1804 is configured to continuously listen for a communication from a glucose meter. A detect operation 1806 determines whether a response is detected by the system 1800. If the detect operation 1806 determines a response is detected, operational flow branches "yes" to a store module 1808. If the detect operation 1806 determines that a response is not detected, operational flow branches "no" to the listen module 1804 such that the system continues to listen for a communication from a glucose meter.

The remainder of system 1800 operates analogously to system 1700 of FIG. 17. The store module 1808 stores the test result associated with the patient data in a memory. In embodiments performed on the monitoring system, the store module 1808 stores the test result in a system memory alongside a patient identification as determined by interfacing with a patient identifier. In embodiments performed on a remote system, the store module 1808 stores the test result in a database such that the test result is accessible to a patient or health care provider at a remote workstation or monitoring system, such as is shown above in FIGS. 3-4.

Once the test result is stored, the actual operational flow of the system 1800 depends upon the component in which the system 1800 operates. In the case of a system 1800 operating in a monitoring system such as is described above in conjunction with FIGS. 3-7, operational flow can optionally be passed to a transmit module 1810. The transmit module 1810 is generally performed in embodiments of the system 1800 resident upon a monitoring system such as the one shown above in FIGS. 3-7. In such embodiments, the transmit module 1810 transmits the test results to the remote system for long-term storage and requests by a patient or health care provider using a monitoring system or workstation.

In the case of a system 1800 operating in a remote system such as is described above in FIGS. 2-4, operational flow proceeds to an alert determination operation 1812. The alert determination operation 1812 accesses data, such as the last test result received by the remote system or historical test result data. Based on the criteria previously described, the alert determination operation 1812 determines whether sending an alert to a health care provider would be appropriate.

If the alert determination operation detects sending an alert would be appropriate, operational flow branches "yes" to an alert generation module 1814. The alert generation module 1814 sends an alert notification to a health care provider, for example a provider at a workstation shown in FIGS. 3-4. The provider can review the patient record and determine what additional action would be appropriate given the specific reasons that the alert was generated. For example, the provider may determine that the patient needs to change their diet or medication regimen.

Operational flow terminates with an end module 1816. Referring back to the alert determination operation 1812, if the alert determination operation 1812 determines that an alert is not appropriate, operational flow branches "no" to the end module 1816, where operational flow terminates.

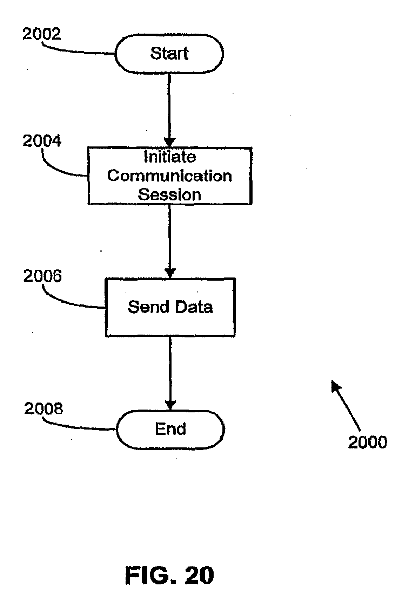

The system 1800 is, in general, particularly configured for operation with glucose meters that alone or in conjunction with communications devices automatically instantiate communication sessions. For example, the system 1800 operates in a complimentary manner to the systems of FIGS. 20-23, below.

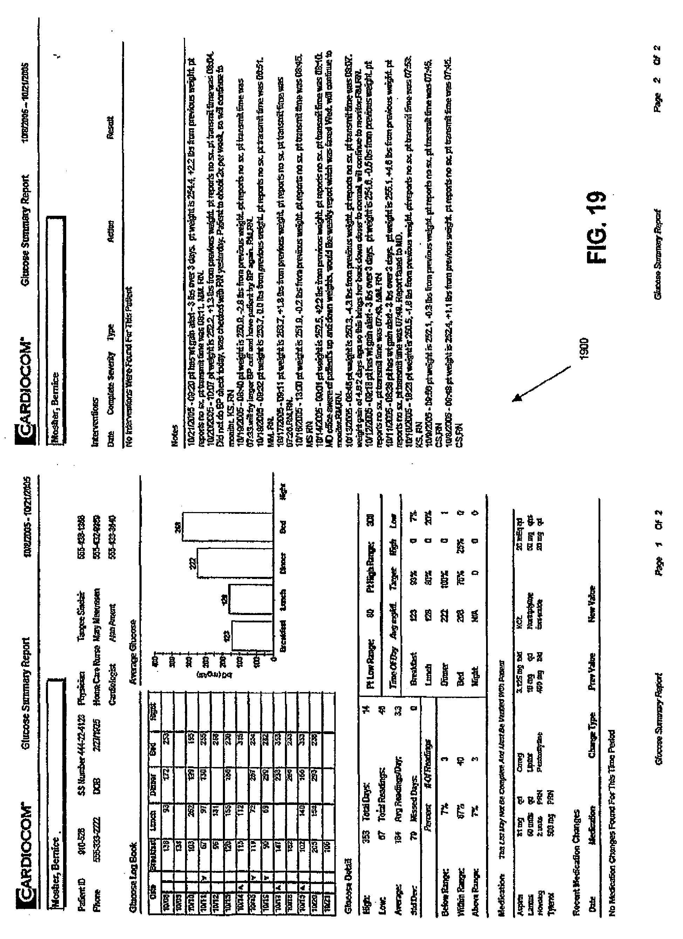

Referring now to FIG. 19, an exception report 1900 is shown that can be generated according to an example embodiment of the present disclosure. The exception report 1900 is one of many alerts that can be created by the systems described above in FIGS. 17-18. The exception report 1900 can be generated, for example, by the remote computing system described above in conjunction with FIG. 2-5. The exception report 1900 can shown current and trended data regarding a given patient, and can describe contributing factors related to a patient's health care regimen, such as medications prescribe, frequency of compliance with blood glucose tests, and historical alerts issued. Of course, additional patient-specific data can be included as well.

The exception report 1900 can take a variety of forms. For example, the exception report can be included in an email message sent to a health care professional or the patient. The exception report can be a file of any user-recognizable format stored on the generating system (i.e. the remote system) or sent to a workstation as shown above in FIGS. 3-4.

Referring now to FIG. 20, a flowchart of systems and methods for communication by a glucose meter is shown according to a possible embodiment of the present disclosure. The system 2000 as shown can be performed by a glucose meter alone, by a glucose meter connected to a communications device such as those described above, or by such a communications device connectable to a glucose meter and constructed to access data held by a glucose meter. The system can be used to maintain constant communicative contact between a glucose meter and a computing system, such as the remote system or monitoring system of FIGS. 2-7.

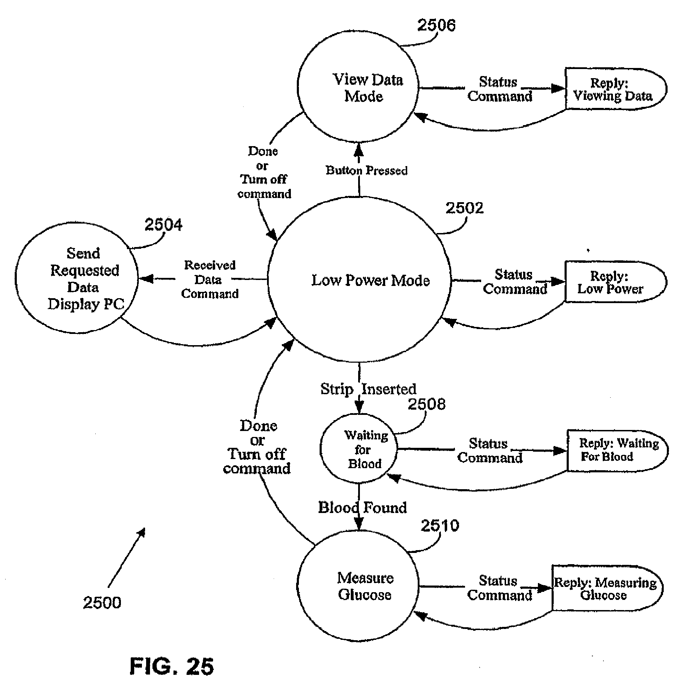

The system 2000 is instantiated by a start module 2002. Operational flow proceeds to an initiation module 2004. The initiation module 2004 begins a communication session with a computing system over a communication link. The initiation module 2004 can be instantiate by a variety of events occurring within a glucose meter communications system. For example, the initiation module 2004 can execute based on a request from a computing system, such as a remote system or monitoring system as described above, that is communicatively connected to the system 2000 via a network link. The initiation module 2004 could also execute automatically at specified intervals or based on a change of mode of the glucose meter, such as between the modes described below in conjunction with FIG. 25. The communication link can include any of a number of wired or wireless connections, and the initiation module can execute based on the system detecting the existence of a communication link.

In one embodiment, the initiation module 2004 instantiates a communication link between the glucose meter and a computing system based on detection of a wired connection to the glucose meter, such as to the computing system or to a communications device such as previously described.

Operational flow proceeds to a send module 2006. The send module 2006 is configured to automatically send data from the glucose meter to the computing system via the communication link. The send module 2006 can send a variety of data from the glucose meter to the computing system, such as the current mode of the glucose meter, a blood glucose test result, a glycosylated hemoglobin test result, or other data representative of a patient's compliance with a blood glucose monitoring regimen.

### Operational flow terminates at an end module 2008.

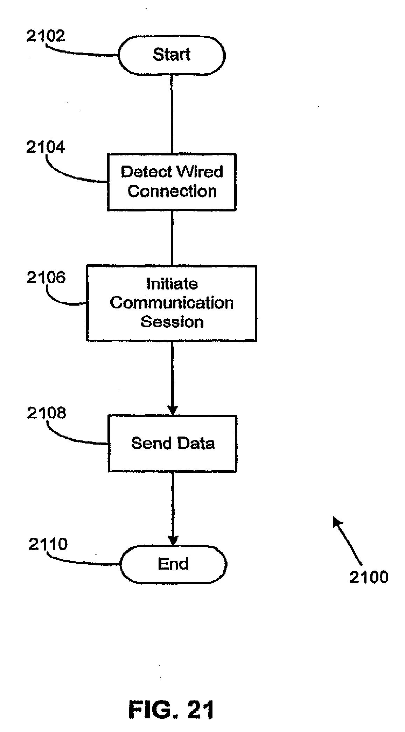

Referring now to FIG. 21, a flowchart of systems and methods for communication by a glucose meter is shown according to a possible embodiment of the present disclosure. The system 2100 can be executed on a glucose meter or a communications device constructed to be interfaced with a glucose meter, such as those described above in conjunction with FIGS. 11-12.

The system 2100 is instantiated by a start module 2102. Operational flow proceeds to a connection detection module 2104. The connection detection module 2104 triggers execution of the system upon detection of a communicative connection between the glucose meter and an external device. In one possible embodiment, the connection is a wired connection between the glucose meter and a communications device such as is described above in conjunction with FIGS. 11-12. Of course, the connection can also be a wired or wireless connection from the glucose meter to a computing system such as the monitoring system or remote system described above in conjunction with FIGS. 2-7.

An initiation module 2106 and a send module 2108 operate analogously to those described in FIG. 20. For example, the data can include a blood glucose test result or a current mode of the glucose meter. The data could also include a message signifying that no blood glucose test result was obtained during the interval, which may indicate a lack of compliance with a blood glucose monitoring regimen.

### Operational flow terminates with an end module 2110.

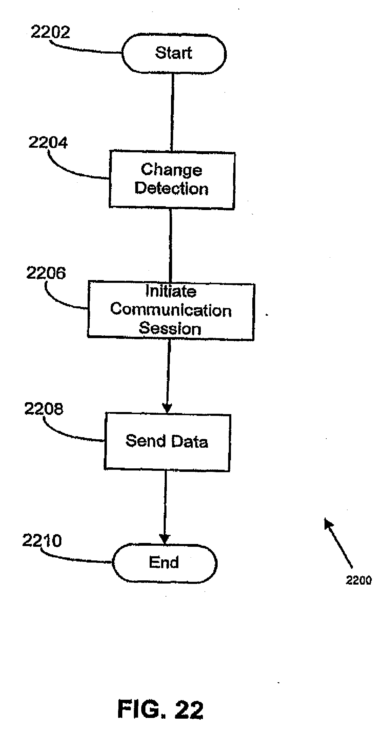

Referring now to FIG. 22, a flowchart of systems and methods for communication by a glucose meter is shown according to another possible embodiment of the present disclosure. The system 2200 can also be executed on a glucose meter or a communications device constructed to be interfaced with a glucose meter, such as those described above in conjunction with FIGS. 11-12.

The system is instantiated by a start module 2202. Operational flow proceeds to a change module 2204. The change module 2204 detects a change in the glucose meter. The change can be, for example, a change between the modes shown below in FIG. 25. Alternately, the change can be an added blood glucose test result available to the glucose meter, such as immediately after a glucose test is performed. In a further embodiment, the change can be a change in time (i.e. a specified interval) determined by the glucose meter.

An initiation module 2206 and a send module 2208 operate analogously to those described in FIG. 20. For example, if a specified interval is detected by the change module 2104, the data sent by the send module could include a new blood glucose test result. The data could also include a message signifying that no blood glucose test result was obtained during the interval, which may indicate a lack of compliance with a blood glucose monitoring regimen. Such a system can interface with the systems described above in FIGS. 17-18 which can receive data from the glucose meter and issue an alert as appropriate.

### Operational flow terminates with an end module 2210.

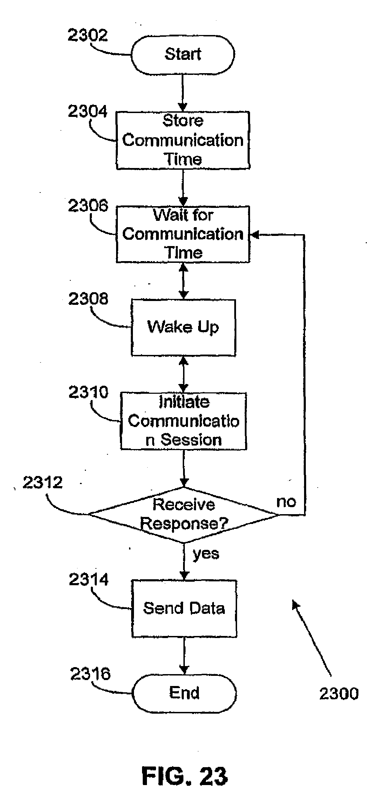

Referring now to FIG. 23, a flowchart of systems and methods for blood glucose monitoring is shown according to a possible embodiment of the present disclosure. The system 2300 as shown can be executed by a glucose meter such as those described above in conjunction with FIGS. 8-16. The system 2300 is configured for periodic communication of glucose meter data to a computing system, such as the remote system and/or monitoring system described above in FIGS. 2-7.

The system 2300 is instantiated by a start module 2302. Following the start module 2302, operational flow proceeds to a timing module 2304. The timing module 2304 allows a user of the glucose meter to program a specific time for the meter to instantiate a communication session with a monitoring system or remote system for the purpose ofuploading test results from blood glucose tests completed by the glucose meter. The timing module 2304 can, for example, allow a user to select times of the day, week, or month to upload results to a specific system or to any available system, depending on the implementation of the communication link between the glucose meter and a computing system, i.e. the remote system or monitoring system.

A wait module 2306 holds the system 2300 in a given state until the predetermined time set in the timing module 2304 occurs. While operational flow resides in the wait module 2306, the system 2300 can exist in a low power or "sleep" state, allowing the system 2300 to conserve power. This functionality is particularly advantageous if system 2300 is operating on a battery-powered device, such as a battery-powered glucose meter.

When the preset time arrives, operational flow proceeds to the wake module 2308 from the wait module 2306. The wake module 2308 activates the various components of the glucose meter in preparation for establishing a communication link to transfer test results from the meter.

An initiation module 2310 sends a communication signal indicating that the glucose meter is seeking to establish a communications session with a monitoring system or remote system. The system 2300 may or may not receive a response from the appropriate responsive computing system (the monitoring system or the remote system), indicating that a communication session is established. However, once the initial signal is sent, the initiation module 2310 passes operational flow to a receive operation 2312.

The receive operation 2312 determines if the system 2300 received a response from an appropriate responsive computing system (the monitoring system or the remote system). If the receive operation 2312 determines that no communication session is established, operational flow branches "no" to the wait module 2306. In this case, the wait module returns the system 2300 to a sleep state until the next communication time occurs. If the receive operation 2312 determines that a communication session is established, operational flow branches "yes" to a send module 2314. The send module 2314 is configured to send data that can include the mode of the glucose meter, or the most recent test results from the glucose meter to the responding computing system.

### Operational flow terminates at end module 2316.

In one particular example of the system 2300, the glucose meter sends daily test result readings to a monitoring system, which in turn stores the readings and sends the readings to a remote computing system in accordance with the methods and systems shown in FIG. 18. In another possible example of the system 2300, the glucose meter sends the test results directly to the remote system.

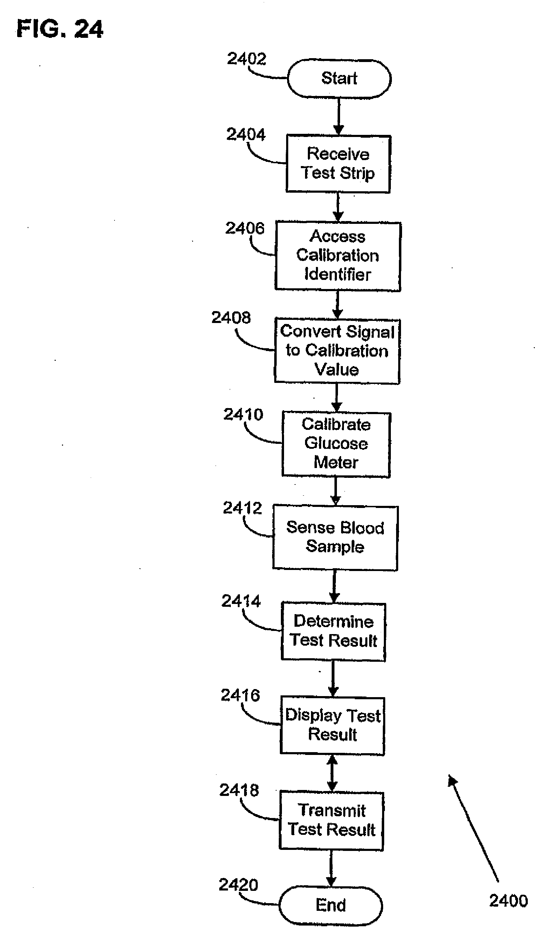

Referring now to FIG. 24, a flowchart of systems and methods for calibration and blood glucose monitoring is shown according to a possible embodiment of the present disclosure. The system 2400 as shown can be executed by a glucose meter such as those described above in conjunction with FIGS. 8-16.

The system 2400 is instantiated by a start module 2402. Following the start module 2402, operational flow proceeds to a receive module 2404. The receive module 2404 includes detecting the receipt of a test strip into a glucose meter, as shown in FIGS. 15-16 above. In various embodiments, the receive module 2404 may include a sensing system for determining when the test strip is sufficiently inserted into the glucose meter.

After the test strip is inserted into the glucose meter, operational flow proceeds to an access module 2406. The access module 2406 accesses a calibration identifier, such as a bar code or integrated circuit, to obtain a code corresponding to the proper calibration of the meter to that test strip. In the case of a bar code embedded on a test strip, the access module 2406 uses an infrared bar code reader to read a bar code located on the test strip inserted into the glucose meter. For example, the access module 2406 could use the sensor shown in FIG. 16 to read a bar code and transmit the bar code sensed to a microcontroller system. In an alternate embodiment where the calibration identifier is an integrated circuit containing an embedded calibration code, the access module 2406 can apply voltage to a lead connected to the integrated circuit so as to access the stored value in the circuit.

Once the access module 2406 reads the calibration identifier present on a test strip, operational flow proceeds to a conversion module 2408. The conversion module 2408 converts the sensed calibration identifier to a numerical value representative of the particular characteristics of the test strip from which the calibration identifier was determined in the access module 2406.

A calibration module 2410 adjusts the calculations or determinations in the glucose meter according to the characteristics of the test strip to ensure accurate results. Specifically, it is often the case that a test strip will have a greater or lesser concentration of reaction chemical on its surface, therefore changing the extent to which a reaction takes place in the test strip that is sensed by the glucose meter. The bar code provides a value to the microcontroller system in the glucose meter to adjust the calculation of blood glucose concentration accordingly so that accurate blood glucose test results are produced.

Once the glucose meter is calibrated, operational flow proceeds to a test module 2412. The test module 2412 detects the concentration of the reaction occurring in the test strip, and a transducer produces an electrical signal representative of the concentration as measured. The electrical signal is passed to a microcontroller system.

A determination module 2414 is configured to produce a numerical value representative of the concentration of glucose in the tested patient's blood based on the electrical signal received from the transducer. The determination module 2414 can calculate or look up the blood glucose value based on the reading sensed in the test strip, and can adjusts the calculation or determination based on the calibration results, which are in turn based on the bar code read from the test strip.

A display module 2416 is configured to display to the patient the numerical representation of the concentration of blood glucose detected in the patient's blood. The display module 2416 may accomplish this by outputting the value to a liquid crystal display, diode display, or other display types capable of communicating the test result to the patient.

After or concurrent with the display module 2416, operational flow proceeds to a transmit module 2418. The transmit module 2418 is configured to transmit data, such as a mode of the glucose meter or blood glucose test results to a monitoring system or remote system consistent with the methods and systems described in conjunction with FIGS. 17-23 and/or 27-28.

### Operational flow terminates at an end module 2420.

The system 2400 can repeat the operation using a second test strip. The second test strip will include a second calibration identifier embodying a second calibration code. By implementing the system 2400, the glucose meter is recalibrated each time a new test strip is inserted.

Referring now to FIG. 25, a flow diagram of a system 2500 for controlling a glucose meter and line-powered communications device is shown according to a further possible embodiment of the present disclosure. The system 2500 described in conjunction with this embodiment can be used in conjunction with any of the systems described above having a line-powered communications device, as in FIGS. 9-10, 14. In the embodiment shown, a default low power mode 2502 is interrupted by received data, a pressed button, or a glucose strip inserted into the glucose meter.

If the system 2500 receives a received data signal, the system 2500 changes state to a data transfer mode 2504. In the data transfer mode 2504, the system 2500 transfers the data via the line-powered communication device to a remote system. When the data transfer operation is completed, the system 2500 returns to the low power mode 2502.

If the system 2500 receives a button pressed signal, the system 2500 changes state to a view data mode 2506. In the view data mode 2506, the glucose meter displays the selected data on a display, such as shown above in conjunction with FIG. 15-16. For example, the data could be the most recent blood glucose test result, or it could include historical test results or additional blood test data. The system 2504 remains in the view data mode 2506 until the glucose meter or line-powered communications device receives a "done" or "turn off" command, upon which the system 2500 returns to the low power mode 2502.

If the system 2500 detects that a glucose test strip is inserted, the system 2500 changes modes to a wait mode 2508. In the wait mode 2508, the system 2500 waits for a user to provide a blood sample on the test strip. Before a blood sample is provided, the system remains in the wait mode 2508.

Once a blood sample is provided, the system 2500 changes state to a measurement mode 2510. In the measurement mode, the system 2500 measures the level of glucose in the blood sample provided on the test strip. This measurement is accomplished consistently with the hardware and software described herein, particularly as in conjunction with FIGS. 8-16. The system remains in the measurement mode 2510 until the glucose meter or line-powered communications device receives a "done" or "turn off" command, upon which the system 2500 returns to the low power mode 2502.

If any other command operation occurs while the system 2500 is in the low power mode 2502, the system 2502 does not change mode.

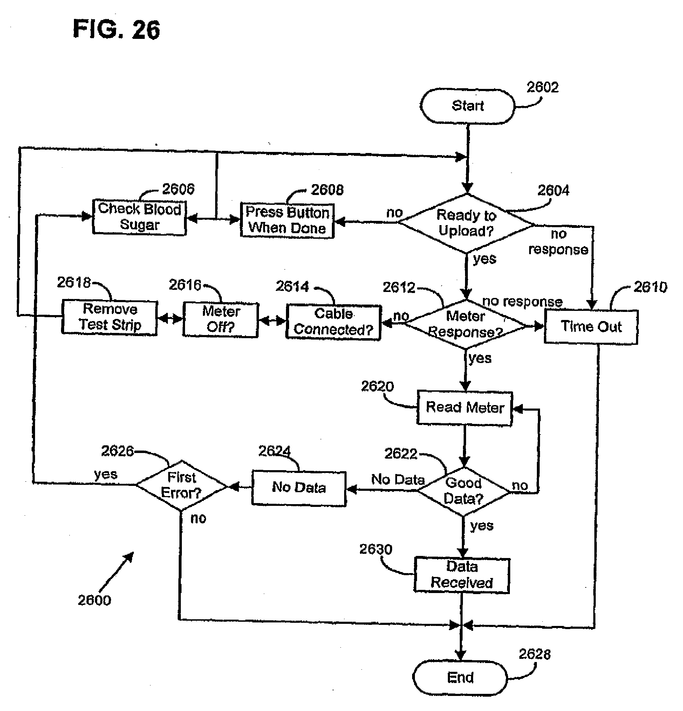

Referring now to FIG. 26, a flow diagram of a data connection system 2600 for use in conjunction with a glucose meter is shown according to a possible embodiment of the present disclosure. The system 2600 can be used in conjunction with a glucose meter connected to either an external line-powered communications device or a monitoring system in an "always on", wired connection, both of which are described in greater detail above.

The system 2600 is instantiated by a start module 2602. Following the start module 2602 operational flow proceeds to an upload operation 2604. The upload operation 2604 determines whether the system 2600 is properly configured to upload test results to a remote system.

If the upload operation 2604 determines that the system 2600 is not prepared to upload data, it is assumed that the glucose meter has not yet completed the blood glucose test, and therefore that results are not yet available to upload. Operational flow branches "no" to a blood glucose test module 2606 and a confirmation module 2608. The blood glucose test module 2606 represents a blood glucose test completed in accordance with the methods described herein. The confirmation module 2608 can be used by a patient to verify that the blood glucose test module 2606 has been completed successfully. When the blood glucose test module 2606 completes and the confirmation module 2608 executes, operational flow branches back to the upload operation 2604.

If the upload operation 2604 determines that the system 2600 does not respond, operational flow branches "no response" to a time out module 2610. The time out module 2610 indicates an unknown failure condition for which the system 2600 will abort attempting to upload data from the glucose meter. Operational flow ends at end module 2628.

If the upload operation 2604 determines that the system 2600 is ready to upload, operational flow branches "yes" to a meter response operation 2612. The meter response operation 2612 determines whether the meter has responded that it is ready to send data to a computing system, such as a remote computing system or a monitoring system as described above. If the meter response module 2612 determines that the meter is not ready, operational flow branches "no" to a series of modules 2614, 2616, 2618 to determine the possible failure condition preventing the system 2600 from establishing such communication. Specifically, a cable connection module 2614 determines whether the cable is properly connected between the glucose meter and either the line-powered communications device or the monitoring system. A meter off module 2616 determines whether the meter is turned off, preventing communication with external devices. A remove test strip module 2618 determines whether a glucose test strip remains connected to the glucose meter operating using system 2600. The remove test strip module 2618 can sense whether a test strip remains connected, and can indicate to the user to remove the strip to allow communication. If none of the modules 2614, 2616, 2618 locate a failure condition or once the modules determine that the failure condition is corrected, operational flow returns to the upload operation 2604. If one of the modules 2614, 2616, 2618 determiners that a failure condition exists, operational flow remains with that module until the error is resolved.

If the meter responses operation 2612 determines that the system 2600 does not respond, operational flow branches "no response" to a time out module 2610. The time out module 2610 indicates an unknown failure condition for which the system 2600 will abort attempting to upload data from the glucose meter. Operational flow again ends at end module 2628.

If the meter response operation 2612 determines that the system 2600 is ready to upload data, operational flow branches "yes" to a read meter module 2620. The read meter module 2620 causes the communication unit, for example the line-powered communications device interfaced with the glucose meter, to access the meter and request the test result representative of the most recent blood glucose level of the patient. This data is sent to the destination computing system, for example the monitoring system or remote system described above.

A data test operation 2622 determines whether the data received from the glucose meter is recognizable as a result of a blood glucose test. If the data test operation 2622 determines that data is not proper, operational flow branches "no" back to the read meter module 2620 to allow the system to retry the communication. If the data test operation 2622 determines that no data is received, operational flow branches "no data" to a no data module 2624, which indicates that an error has occurred. An error counting operation 2626 determines whether the error that occurred is the first error. If the error counting operation 2626 determines that the error is the first error, operational flow branches "yes" back to the blood glucose test module 2606 and confirmation module 2608 to retry the blood glucose test. Upon completion and confirmation of the blood glucose test, operational flow proceeds to the upload module 2604. If the error counting operation 2626 determines that the error is not the first error, operational flow branches "no and the system terminates operation at an end module 2628.

Referring back to the data test operation 2622, if the data test operation 2622 determines that the data received is good, operational flow branches "yes" to a data received module 2630. The data received module 2630 can confirm receipt of the test result, and can store the test result in a memory of the computing system. In particular embodiments, the test result is associated with an identifier of a patient, allowing the system 2600 to track the blood glucose test results of multiple patients.

### Operational flow terminates at the end module 2628.

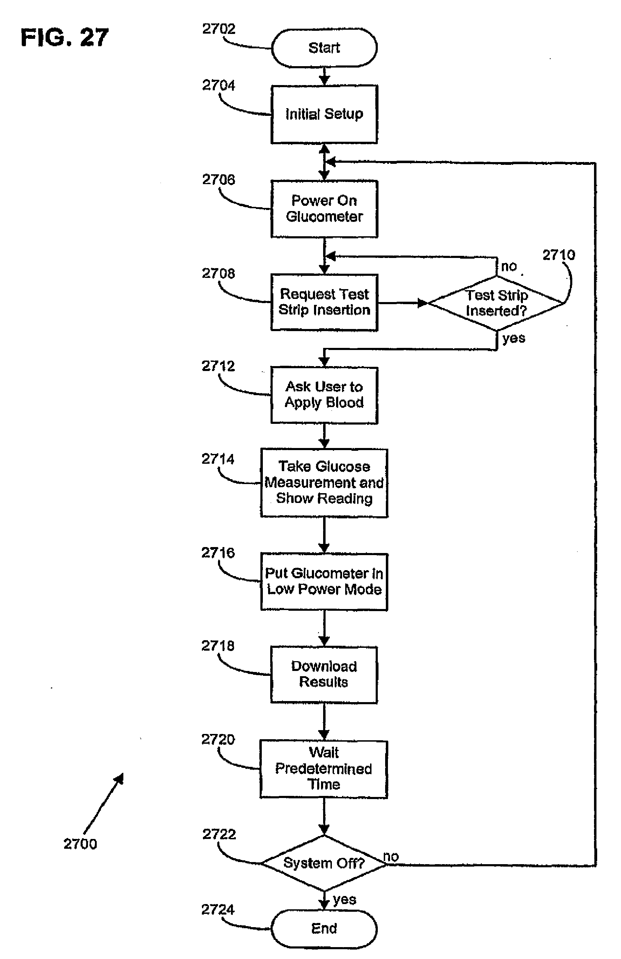

Referring now to FIG. 27, a system for glucose meter communication is shown according to a further possible embodiment. The system 2700 as shown is particularly applicable to instances where the glucose meter is communicatively connected or integral with a line-powered communications device, such as a line-powered modem, that is configured to selectively power the glucose meters the embodiment shown, the line-powered communications device is in an "always connected" mode, which means that the communications device remains in communicative connection with a requesting computing device such as the remote system or monitoring system described above. The system 2700 is instantiated by a start module 2702.

A setup module 2704 performs the initial operations required to establish communication with a separate computing system, such as the remote system or monitoring system described above.

A power module 2706 sends a signal to the glucose meter, causing the glucose meter to turn on. For example, the power module 2706 could provide a power signal to the glucose meter, or could activate an electronic or electromechanical switch causing the glucose meter to turn on.

A request module 2708 communicates with a user of the system 2700, such as a patient that is using the glucose meter. The request module 2708 indicates to the user/patient that a glucose test strip should be inserted into the glucose meter.

A test strip detection operation 2710 determiners whether a test strip has been inserted. For example, the test strip detection operation 2710 can determine if the incorrect type of test strip is inserted into the glucose meter, or whether a test strip is being inserted incorrectly, or other incorrect use. If the test strip operation 2710 determines that a test strip has not been inserted correctly, operational flow branches "no" to the request module 2708. If the test strip operation 2710 determines that a test strip has been inserted correctly, operational flow branches "yes" to a blood sample module 2712. The blood sample module 2712 requests a blood sample be applied to the test strip so that the glucose meter can derive a blood glucose test result.

A measurement module 2714 computes the blood glucose test result based on the blood sample applied to the test strip in the blood sample module 2712. The measurement module 2714 also displays the results of the blood glucose test on a display, such as the one discussed above in conjunction with FIGS. 15-16.

A low power module 2716 causes the system 2700 to place the glucose meter in a low power mode, as described in conjunction with FIG. 25.

A download module 2718 transfers the test result as computed by the glucose meter to a separate computing system via a communication link, such as the remote system or monitoring system described above. The download module 2718 can initiate a communication session between a remote system and a glucose meter or communications device wired to the glucose meter prior to transferring the test result.

A wait module 2720 holds the system 2700 in an idle state for a predetermined time. The wait module 2720 can hold the system 2700 in the idle state for any amount of time, or can be programmable/selectable by either a patient or health care provider. In one possible example of the present disclosure, the wait module 2720 waits 12 hours, coinciding with a twice daily blood glucose test. Of course, other time periods can be implemented as well.

A power operation 2722 determines whether the system is turned off following the downloading of test results. If the power operation determines that the power is not turned off, operational flow proceeds to the power on module 2706 so that the system 2700 can repeat the downloading of test results once the wait module 2720 has completed. If the power operation 2722 determines that the power is off, operational flow is terminated at an end module 2724.

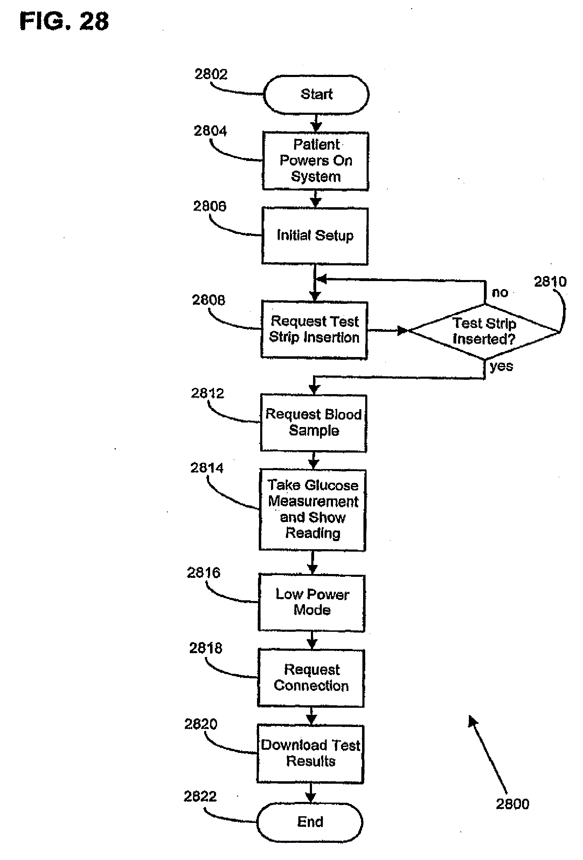

Referring now to FIG. 28, a system for glucose meter communication is shown according to a further possible embodiment. The system 2800 as shown is also applicable to instances where the glucose meter is communicatively connected or integral with a line-powered communications device, such as a line-powered modem, that is configured to selectively power the glucose meter. In the embodiment shown, the line-powered communications device is in a "power save" mode, which means that the communications device does not remain in communicative connection with a requesting computing device, and instead requires user intervention for downloading results.

The system 2800 is instantiated by a start module 2802. In a power module 2804, a user, such as a patient, powers on the system 2800. This can be accomplished, for example, by simply pressing a power button on the glucose meter and, if present, the separate line-powered communication device.

A setup module 2806 initializes the system 2800 by setting any required variables and, if the glucose meter is separate from the line-powered communication device, initializing a communication session between the separate units.

A request module 2808 communicates with a user of the system 2800, such as a patient that is using the glucose meter. The request module 2808 indicates to the user/patient that a glucose test strip should be inserted into the glucose meter.

A test strip detection operation 2810 determines whether a test strip has been inserted. For example, the test strip detection operation 2810 can determine if the incorrect type of test strip is inserted into the glucose meter, or whether a test strip is being inserted incorrectly, or other incorrect use. If the test strip operation 2810 determines that a test strip has not been inserted correctly, operational flow branches "no" to the request module 2805. If the test strip operation 2810 determines that a test strip has been inserted correctly, operational flow branches "yes" to a blood sample module 2812. The blood sample module 2812 requests a blood sample be applied to the test strip so that the glucose meter can derive a blood glucose test result.

A measurement module 2814 is included in the system 2800, and computes the blood glucose test result based on the blood sample applied to the test strip in the blood sample module 2812. The measurement module 2814 also displays the results of the blood glucose test on a display, such as the one discussed above in conjunction with FIGS. 15-16.

In a low power module 2816, the system 2800 places the glucose meter in a low power mode in order to conserve the battery life of the glucose meter. A connection module 2818 requests a connection between the communications device and a computing system such as the remote system or monitoring system above. When a connection is established, operational flow proceeds to a download module 2820. The download module 2820 transfers the test result as computed by the glucose meter to a separate computing system via a communication link, such as the remote system or monitoring system described above.

The system terminates at an end module 2822.

Aspects of the invention described as being carried out by a computing system or are otherwise described as a method of control or manipulation of data may be implemented in one or a combination of hardware, firmware, and software. Embodiments of the invention may also be implemented as instructions stored on a machine-readable medium, which may be read and executed by at least one processor to perform the operations described herein. A machine-readable medium may include any mechanism for storing or transmitting information in a form readable by a machine (e.g., a computer). For example, a machine-readable medium may include read-only memory (ROM), random-access memory (RAM), magnetic disc storage media, optical storage media, flash-memory devices, electrical, optical, acoustical or other form of propagated signals (e.g., carrier waves, infrared signals, digital signals, etc.), and others.

In the forgoing detailed description, various features are occasionally grouped together in a single embodiment for the purpose of streamlining the disclosure. This method of disclosure is not to be interpreted as reflecting an intention that the claimed embodiments of the subject matter require more features than are expressly recited in each claim. Rather, as the following claims reflect, inventive subject matter lies in less than all features of a single disclosed embodiment. Thus, the following claims are hereby incorporated into the detailed description, with each claim standing on its own as a separate preferred embodiment. Therefore, the spirit and scope of the appended claims should not be limited to the description of the preferred versions contained herein.

Further Embodiments:
1. A method of communicating data between a glucose meter and a computing system, the method comprising:
   automatically initiating a communication session between the glucose meter and the computing system over a communication link;
   automatically sending data from the glucose meter to the computing system via the communication link.
2. The method of item 1, further comprising:
   (a) physically connecting the glucose meter to a communications device.
3. The method of item 2, wherein:
   (a) automatically initiating a communication session occurs in response to physically connecting the glucose meter to the communications device.
4. The method of item 1, further comprising:
   (a) storing a predetermined communication time;
5. The method of item 4, wherein;
   (a) automatically initiating the communication session occurs based upon the predetermined communication time.
6. The method of item 1, further comprising:
   (a) receiving a response from the computing system.
7. The method of item 1, wherein:
   (a) automatically sending data comprises sending a test result.
8. The method of item 7, wherein:
   (a) sending a test result comprises sending data representative of a blood glucose test result.
9. The method of item 1, wherein:
   (a) automatically sending data comprises sending data representative of a mode of the glucose meter.
10. The method of item 1, wherein:
   (a) automatically initiating includes initiating a communication session with a remote system.
11. The method of item 1 wherein:
   (a) automatically initiating includes initiating a communication session with a monitoring system.
12. The method of item 1, further comprising:
   (a) waking the glucose meter from a low power state.
13. The method of item 12, further comprising:
   (a) obtaining a blood glucose test result after waking the glucose meter.
14. The method of item 12, further comprising:
   (a) viewing data displayed by the glucose meter after waking the glucose meter.
15. A method of gathering and communicating test results with a glucose meter, the method comprising:
   storing a predetermined communication time;
   initiating the glucose meter to enter a low power mode;
   waiting for the predetermined communication time with the glucose meter in the low power mode;
   upon reaching the predetermined communication time, initiating the glucose meter to exit the low power mode;
   automatically initiating a communication session with a computing system via a communication link at the predetermined communication time; and
   sending data to the computing system via the communication link.
16. The method of item 15, further comprising:
   (a) receiving a response from the computing system.
17. The method item 15, further comprising:
   sensing a blood glucose sample in the glucose meter; and
   determining a test result comprising a blood glucose test result;
   wherein the data comprises the test result.
18. The method of item 17, further comprising:
   (a) displaying the test result on a display integrated into the glucose meter.
19. The method of item 17, wherein:
   (a) determining a test result includes calculating a test result representative of a glycosylated hemoglobin level of a patient.
20. The method of item 15, further comprising:
   (a) detecting one or more failure conditions of the glucose meter prior to automatically initiating a communication session.
21. The method of item 15, wherein:
   (a) determining a test result comprises calculating a test result representative of a blood glucose level of a patient.
22. The method of item 15, wherein:
   (a) automatically initiating a communication session is accomplished by a communication device incorporated into the glucose meter.
23. The method of item 15, wherein:
   (a) automatically initiating a communication session is accomplished by a communication device separate from the glucose meter.
24. A method of obtaining blood glucose test results from a glucose meter, the method comprising:
   monitoring a communication link for a signal from the glucose meter at a given frequency;
   detecting the existence of the signal from the glucose meter via the communication link;
   initiating communication with the glucose meter via the communication link; and
   receiving data From the glucose meter via the communication link.
25. The method of item 24, further comprising:
   (a) sending a request to the glucose meter before listening for a signal.
26. The method of item 25, further comprising:
   (a) waiting a predetermined time after detecting no response before sending another request.
27. The method of item 26, wherein:
   (a) the given frequency is dependent upon the predetermined time of the waiting step.
28. The method of item 25, wherein:
   (a) sending a request includes transmitting a request over a wireless network.
29. The method of of item 24, wherein:
   (a) monitoring a communication link includes sensing a wireless signal.
30. The method of of item 24, further comprising:
   (a) storing a response from the glucose meter in a computing system, the response including a test result.
31. The method of item wherein:
   (a) storing the response includes storing a blood glucose level.
32. The method of item 30, wherein:
   (a) storing the response includes storing a glycosylated hemoglobin level.
33. The method of of item 30, wherein:
   (a) storing the response includes storing a patient identifier associated with the test result.
34. The method of of item 29, wherein:
   (a) storing the response includes storing a device identifier associated with the test result.
35. The method of item 24, further comprising:
   tracking the glucose level of a patient; and
   providing an alert based upon the glucose level.
36. A system for coordinating communication of blood glucose test results comprising:
   (a) a glucose meter configured to obtain a test result representative of a blood glucose level of a patient;
   (b) a remote system configured to communicate with the glucose meter via a network, the remote system configured to receive and store test results obtained by the glucose meter in a database containing patient data;
   (c) wherein the remote system periodically receives the test results via a communication session automatically initiated by a component of the system; and
   (d) wherein the remote system tracks the glucose level of the patient and generates an alert based upon the glucose level of the patient.
37. The system of item 36, wherein:
   (a) the remote system alerts a health care provider if the test result is outside the parameters so that the health care provider can contact the patient.
38. The system of item 36, wherein:
   (a) the alert comprises an exception report.
39. The system of item 36, further comprising:
   (a) a monitoring system configured to communicate with the glucose meter and configured to receive and store test results obtained by the glucose meter.
40. The system of item 36, further comprising:
   (a) a plurality of glucose meters configured to communicate with a plurality of monitoring systems, the plurality of monitoring systems configured to communicate with the remote system via the network.
41. The system of item 36, further comprising:
   (a) a workstation configured to communicate with the remote system via the network, the workstation configured to access at least a portion of the patient data stored in the database.
42. The system of item 36, further comprising:
   (a) a line-powered communications device configured to communicate with the glucose meter.
43. The system of item 36, wherein:
   (a) the component of the system is the monitoring system.
44. The system of of item 36 wherein:
   (a) the component of the system is the remote system.
45. The system of item 36, wherein:
   (a) the component of the system is the glucose meter.
46. The system of item 42, wherein:
   (a) the component of the system is the line-powered communications device.
47. The system of item 36, wherein:
   (a) the test result includes data representative of a blood glucose test result.
48. The system of item 36, wherein:
   (a) the test result includes data representative of a glycosylated hemoglobin test result.
49. A glucose meter system comprising:
   (a) a glucose meter configured to determine a test result representative of a patient's blood glucose level;
   (b) a line powered communication device communicatively coupled to the glucose meter, the line powered communication device configured to access data and communicate the data over a wired communication link, the data including the test result, wherein the line powered communications device is at least partially powered with power received from the wired communication link.
50. The glucose meter system of item 49, wherein:
   (a) the line powered communication device is configured to selectively place the glucose meter in a low power mode and remove the glucose meter from the low power mode.
51. The glucose meter system of item 49, wherein:
   (a) the line powered communication device resides within a housing integral with the glucose meter.
52. The glucose meter system of item 49, wherein:
   (a) the line powered communication device is a line powered modem.
53. The glucose meter system of item 49, wherein:
   (a) the line powered communication device provides a power signal to the glucose meter.
54. The glucose meter system of item 49, further comprising:
   (a) a control signal configured to allow a patient to selectively power the glucose meter system with a battery or the line powered communication device.
55. The glucose meter system of item 49, wherein:
   (a) the glucose meter is operable in a low power mode wherein the line powered communication device remains active and can access the test result in the glucose meter while the glucose meter is in the low power mode.
56. The glucose meter system of item 49, wherein:
   (a) the line powered communication device communicates the test result to a remote system via the communications link.
57. The glucose meter system of item 49, wherein:
   (a) the communication link comprises a network connection.
58. The glucose meter system of item 49, wherein:
   (a) the communication link comprises a telephone line.
59. The glucose meter system of item 49, wherein:
   (a) the line powered communication device maintains a communicative connection to a remote system via the communication link.
60. A blood glucose test result communication system comprising:
   (a) a glucose meter configured to compute and store a test result representative of a patient's blood glucose level;
   (b) a line powered communication device communicatively coupled to the glucose meter and configured to automatically access the test result and communicate the test result over a wired communication link, the line powered communication device at least partially powered with power received from the wired communication link;
   (c) a remote system communicatively connected to the line powered communication device via the communication link, the remote system configured to accept and store the test result in a database.
61. The blood glucose test result communication system of item 60, wherein:
   (a) the line powered communication device resides within a housing integral with the glucose meter.
62. The blood glucose test result communication system of item 60, wherein:
   (a) the line powered communication device is a line powered modem.
63. The blood glucose test result communication system of item 60, wherein:
   (a) the line powered communication device is configured tor communicate the test result to the remote system at a predetermined time.
64. The blood glucose test result communication system of item 60, wherein:
   (a) the communication link is a network connection.
65. The blood glucose test result communication system of item 60, wherein:
   (a) the glucose meter includes a low power mode.
66. The blood glucose test result communication system of item 60, wherein:
   (a) the line powered communication device is configured to communicates the test result over a communication link while the glucose meter resides in the low power mode.
67. The blood glucose test result communication system of item 60, wherein:
   (a) the line powered communication device is configured to communicate directly with the remote system via the communication link.
68. The blood glucose test result communication system of item 60, wherein:
   (a) the database includes test results from a plurality of glucose meters received by the remote system.
69. The blood glucose test result communication system of item 68, wherein:
   (a) the database associates the test results with one or more patient identifiers.
70. A method of communicating between a glucose meter and a remote system, the glucose meter communicatively connected to a line powered communication device at least partially powered with power received from a wired communication link, the method comprising:
   acquiring a test result representative of a blood glucose level, the test result accessible to the glucose meter and the line powered communication device;
   placing the glucose meter in a low power mode; and
   transferring the test result from the line powered communication device to the remote system.
71. The method of item 70, further comprising:
   (a) initiating a connection between the line powered communications device and the remote system.
72. The method of item 71, wherein:
   (a) initiating a connection between the line powered communication device and the remote system is performed by the line powered communication device:
73. The method of item 70, wherein:
   (a) the test result is based on a blood sample applied to a test strip.
74. The method of item 70, further comprising:
   powering on the glucose meter;
   initializing the line powered communication device;
   requesting insertion of a test strip into the glucose meter; and
   requesting a blood sample be applied to the test strip.
75. The method of item 74, wherein:
   (a) powering on the glucose meter is performed by the line powered communications device.
76. The method of item 74, wherein:
   (a) powering on the glucose meter is performed by a patient using the glucose meter.
77. The method of item 74, wherein:
   (a) initializing the line powered communication device is performed before powering on the glucose meter.
78. The method of item 70, further comprising:
   (a) displaying the test result on a display integrated into the glucose meter.
79. The method of item 70, further comprising:
   (a) waiting a predetermined time and repeating one or more steps so as to transfer a second test result from the line powered communication device to the remote system.
80. The method of item 70, wherein:
   (a) transferring the test result comprises automatically transferring the test result to the remote system.
81. A method of communicating a blood glucose test result between a glucose meter and a computing system, the method comprising:
   waking the glucose meter from a low power state;
   obtaining a blood glucose test result;
   storing a predetermined communication time;
   physically connecting the glucose meter to a communications device;
   placing the glucose meter in the low power state;
   automatically initiating a communication session between the glucose meter and the computing system over a communication link at the predetermined communication time and in response to physically connecting the glucose meter to the communications device;
   receiving a response from the computing system; and
   automatically sending the blood glucose test result from the glucose meter to the computing system via the communication link.

## Claims

1. A blood glucose test result communication system comprising:
(a) a glucose meter configured to compute and store a test result representative of a patient's blood glucose level;
(b) a line powered communication device communicatively coupled to the glucose meter and configured to automatically access the test result and communicate the test result over a wired communication link, the line powered communication device at least partially powered with power received from the wired communication link;
(c) a remote system communicatively connected to the line powered communication device via the communication link, the remote system configured to accept and store the test result in a database.

2. The blood glucose test result communication system of claim 1, wherein:
(a) the line powered communication device resides within a housing integral with the glucose meter.

3. The blood glucose test result communication system of claim 1, wherein:
(a) the line powered communication device is a line powered modem.

4. The blood glucose test result communication system of claim 1, wherein:
(a) the line powered communication device is configured to communicate the test result to the remote system at a predetermined time.

5. The blood glucose test result communication system of claim 1, wherein:
(a) the communication link is a network connection.

6. The blood glucose test result communication system of claim 1, wherein:
(a) the glucose meter includes a low power mode.

7. The blood glucose test result communication system of claim 1, wherein:
(a) the line powered communication device is configured to communicate the test result over a communication link while the glucose meter resides in the low power mode.

8. The blood glucose test result communication system of claim 1, wherein:
(a) the line powered communication device is configured to communicate directly with the remote system via the communication link.

9. The blood glucose test result communication system of claim 1, wherein:
(a) the database includes test results from a plurality of glucose meters received by the remote system.

10. The blood glucose test result communication system of claim 9, wherein:
(a) the database associates the test results with one or more patient identifiers.

11. A method of communicating between a glucose meter and a remote system, the glucose meter communicatively connected to a line powered communication device at least partially powered with power received from a wired communication link, the method comprising:
acquiring a test result representative of a blood glucose level, the test result accessible to the glucose meter and the line powered communication device;
placing the glucose meter in a low power mode; and
transferring the test result from the line powered communication device to the remote system.

12. The method of claim 11, wherein:
(a) initiating a connection between the line powered communication device and the remote system by the line powered communication device,

13. The method of claim 11, wherein:
(a) the test result is based on a blood sample applied to a test strip,

14. The method of claim 11, further comprising:
powering on the glucose meter;
initializing the line powered communication device;
requesting insertion of a test strip into the glucose meter; and
requesting a blood sample be applied to the test strip.

15. The method of claim 11, further comprising:
(a) waiting a predetermined time and repeating one or more steps so as to transfer a second test result from the line powered communication device to the remote system.
